# EUROPEAN PATENT SPECIFICATION

(11) **EP 0 701 839 B1**
(45) Date of publication and mention of the grant of the patent: **23.05.2001**
(21) Application number: 95112617.6
(22) Date of filing: 10.08.1995
(51) Int. Cl.: A61N 5/06

(54) **Therapeutic products containing organic ashes emitting 'far' infrared radiation**
Therapeutische Produkte mit organischen Aschen, welche Ferninfrarotstrahlen emitieren
Produits thérapeutiques contenant des cendres organiques émettant dans l'infra-rouge lointain

(30) Priority: 11.08.1994 JP 21180294; 01.08.1995 JP 21674695
(43) Date of publication of application: 20.03.1996
(73) Proprietor: Hori, Yasunori, Yokkaichi-Shi, Mie-Ken (JP)
(72) Inventor: Hori, Yasunori, Yokkaichi-Shi, Mie-Ken (JP)
(74) Representative: Jung, Elisabeth, Dr.

(56) References cited:
- EP-A- 0 417 789
- EP-A- 0 489 538

## Description

### Background of the Invention

This invention relates to therapeutic products such as ointments, cataplasm poultices, accessories, ceramics, paints, paper and cloth materials, resins and energy-saving construction materials having ashes of organic origins mixed therewith and being capable of promoting health and healing patients by the heating effects of far infrared radiation emitted therefrom.

Examples of material with the property of emitting far infrared radiation include ceramics, alumina and silica. Ceramics, for example, have emissivity of about 60% under a high-temperature condition of about 500°C - 700°C. Far infrared radiation is in the wavelength range of 4.0 - 1000µm. Although its photon energy is smaller than that of near infrared radiation, far infrared radiation has stronger transmissivity and can reach and effectively heat the interior of an organism with only a small temperature increase on the surface. It is known that far infrared radiation in the wavelength range of 8 - 12µm is particularly effective to a human body. If the emission temperature of far infrared radiation is as high as 500°C - 700°C, however, its usefulness is limited.

It was reported recently that carbon has the largest far infrared radiation emissivity of about 90% among various materials within the temperature range of 27°C to 227°C. Carbon fibers produced by pulverizing carbon into fine particles and mixing them with ordinary fibers have also been reported. A rise in the body temperature by as much as 1.25°C has been ascertained of a person who wore clothes made of such a fiber material, and such fiber products are about to be made available commercially. It is also known that far infrared radiation effects are obtained if a paint mixed with seaweed charcoal is applied.

Silicone resin paints capable of emitting far infrared radiation using metal oxides such as copper oxide have also been known, but they are costly. Materials emitting far infrared radiation at high temperatures about 100°C are not practical and not suited as a building material. Although building materials for residential homes are required to have heat-insulating property in addition to light-dimming, sound-absorbing and decorative properties, there is a limit to their heat-insulating capability. Although an air conditioner may be used to adjust the indoor temperature at a fixed level, the indoor temperature is easily influenced by the temperature outdoors, and it is difficult to maintain the temperature at a constant level. In other words, the cooling and heating effects of presently available materials are not very good.

Since seaweed charcoal contains a large amount of carbon as shown in Table 1 and is black, it is difficult to use when it is desired to obtain a product having a specified color other than black. In general, when a carbon-containing coloring agent is used for the purpose of making anything black, such as resin, only a few percent of carbon is usually enough for the purpose. This means that, if seaweed charcoal is added even by a small amount, the mixture can only be very black, or that the color of anything that contains seaweed charcoal is limited to black. If an ointment mixed with seaweed charcoal is applied on a skin part, for example, the applied area of the skin will turn black, and this black color will begin to flow as the sweating is accelerated by the far infrared effect. Moreover, black stains by seaweed charcoal are difficult to wash.

**Table 1**

| Element | Content |
|---|---|
| Carbon | 41.5% |
| Potassium | 7.02% |
| Sodium | 3.41% |
| Magnesium | 2.61% |
| Iron | 3.15% |
| Calcium | 12.5% |
| Chlorine | 8.25% |
| Manganese | 589ppm |
| Nickel | 275ppm |
| Zinc | 195ppm |
| Copper | 30.2ppm |
| Lead | 3.2ppm |
| Chromium | 1350ppm |
| Silicon | 1.5% |
| Aluminum | 2.74% |
| Lithium | 4.8ppm |
| Iodine | 0.028% |
| Phosphorus | 0.091% |
| Sulphur | 1.42% |
| Strontium | 1800ppm |

Another problem to be considered is that ill effects of micro-particles of carbon and its compounds on human body are being reported. Since wood charcoal and seaweed charcoal contain micro-particles of carbon, ill effects of their use when they are contacted with or rubbed into the skin may be very serious. In fact, many deaths by skin cancer caused by soot among chimney sweeps were reported in Europe. It may be considered that the many instances of skin cancer were due to a combined effect of carbon or its compounds with heat energy. Even now, the rate of occurrence of skin cancer is said to be high among people who work with soot such as makers of ink-sticks, but it is reported that the rate of occurrence among them is much lower probably because there is no element of heat in the case of ink-stick makers. This tends to prove that a direct contact of the body with carbon or its compounds, together with application of heat, is a likely cause of skin cancer. In other words, it may be considered safer to avoid simultaneously using an emitter of far infrared radiation and carbon on a body.

Still another problem is that it is difficult to mix wood charcoal or seaweed charcoal into a metal, glass or a ceramic material since they contain a large amount of carbon which burns at the time of the mixing at the temperature of molding. In order to efficiently obtain wood charcoal or seaweed charcoal of a high quality, furthermore, a special oven is required such that temperature and air supply can be controlled. Since the quality of the product depends sensitively on the control of conditions during the burning process, the production cost becomes high.

### Summary of the Invention

This invention has been accomplished in order to eliminate these problems of prior art technologies, and its object is to provide an emitter of far infrared radiation which can be produced inexpensively and does not have the problem of color.

It is another object of this invention to provide therapeutic products such as ointments and cataplasm poultices, adhesive tapes, accessories, health items and equipment, glass, pottery, paper and cloth materials, paints and resins containing such a far infrared emitter and also energy-saving construction materials with good cooling and warming effects.

To accomplish the objects described above, the present invention teaches the use of organic ashes as a source of far infrared radiation at human body temperatures. Ashes of organic origin such as ashes of animals, inclusive of birds, fishes and shells, parts thereof and their excrements, ashes of woods, grasses, plants and grains, as well as their products such as old newspapers and magazines, and exclusive of radioactive ashes from a nuclear reactor, will be referred to as "organic ashes" throughout herein. For example, this invention relates to natural and polymer resins containing organic ashes as a far infrared radiation emitter by at least 0.01% to be used for making containers, electrical home appliances, containers for dental equipments, containers for cosmetic materials and many kinds of health items.

The invention also relates to sheets and films containing organic ashes as a far infrared radiation emitter by at least 0.01% to be used for making vinyl sheets for household use and water-proof sheets.

This invention also relates to coating materials of all kinds such as oil-based paints, water-based paints, coloring paints, artist's paints, fluorescent paints and household paints containing organic ashes as a far infrared radiation emitter by 0.01% to 90%.

This invention also relates to health-promoting construction materials such as heat insulators, wall papers, cloth products and materials, cement and mortar containing organic ashes as a far infrared radiation emitter by 0.01% to 50%.

This invention also relates to medical products such as ointments and medical equipment containing organic ashes as a far infrared radiation emitter by 0.01% to 30%.

This invention also relates to cataplasm poultices having organic ashes as a far infrared radiation emitter mixed with a medicament by 0.01% to 20%.

This invention also relates to tapes for medical applications such as emergency adhesive tapes and cloth-backed tapes as well as tapes for office use and for packaging, containing organic ashes as a far infrared radiation emitter by 0.01% to 50%.

This invention also relates to health items combining a mixture of a magnetic healing product and organic ashes with an adhesive tape.

This invention also relates to accessories such as rings and bracelets made of a material containing organic ashes as a far infrared radiation emitter by 0.01% to 90%.

This invention also relates to hygienic items made of a material containing organic ashes as a far infrared radiation emitter by 0.01% to 30%.

This invention also relates to household items including dinnerware and paper products containing organic ashes as a far infrared radiation emitter by 0.01% to 30%.

This invention also relates to porcelain housewares with a glaze containing organic ashes as a far infrared radiation emitter by 0.01% to 100%.

This invention also relates to enamels containing organic ashes as a far infrared radiation emitter by 0.01% to 50%.

This invention also relates to household glass products such as glass dinnerware, thermos bottles and window panes containing organic ashes as a far infrared radiation emitter by 0.01% to 70%.

This invention also relates to beauty and cosmetic products such as hair washes, cosmetic liquids and packs containing organic ashes as a far infrared radiation emitter by 0.01% to 40%.

This invention also relates to health-promoting clothing items such as underclothes and garments made of a cloth material containing organic ashes as a far infrared radiation emitter by 0.01% to 50% in the fibers or in the coating material which is spread over them.

This invention also relates to furniture made of a material containing organic ashes as a far infrared radiation emitter by 0.01% to 30%.

With products according to this invention thus containing organic ashes as defined above, the heat energy of body temperature of a person in contact therewith or in the vicinity thereof can be converted at a high efficiency of about 90% into far infrared radiation which is a kind of electromagnetic waves. The far infrared radiation serves to raise the body temperature, and this energy is again converted into far infrared radiation. This invention provides materials which make this repeated processes possible, and desirable results such as improved blood circulation, raised peripheral body temperature and increased rate of alpha-wave generation are obtained by using products made with such materials. The materials to which organic ashes are to be added according to this invention include wood materials, resins, paper, cloth fibers, metals, medicaments, raw materials for porcelain and paints and glass materials.

### Brief Description of the Drawings

The accompanying drawings, which are incorporated in and form a part of this specification, illustrate embodiments of the invention and, together with the description, serve to explain the principles of the invention. In the drawings:
Figs. 1 through 6 are radiation intensity spectra of ashes respectively of seaweeds, plants, newspapers, sardines, pork and defatted soy beans, used according to this invention;
Figs. 7 through 12 are radiation emissivity of ashes respectively of seaweeds, plants, newspapers, sardines, pork and defatted soy beans;
Figs. 13-17 are graphs showing the changes in the tensile strength respectively of high-density polyethylene (HDPE), polypropylene (PP) resin, hard vinyl chloride resin, polyethylene terephthalate (PET) resin and acrylonitrile butadiene styrene (ABS) resin as their content of newspaper ashes is increased;
Figs. 18-21 are graphs showing the changes in the adhesion strength of coating materials respectively comprising vinyl chloride resin (with molecular weight of 50000), aminoalkyd resin, unsaturated polyester resin and acryl lacquer as their content of newspaper ashes is increased;
Figs. 22-25 are graphs showing changes in body temperature of patients, indicative of effects of the present invention.
Fig. 26 is a graph showing the change in the elongation of a resin fiber according to its ash content;
Fig. 27 is a graph showing the change in the tensile strength of a ceramic material according to its ash content; and
Fig. 28 is a graph showing the change in cluster size of tap water placed inside containers according to this invention.

### Detailed Description of the Invention

The invention is described below by way of examples. To start, methods of producing organic ashes to be mixed according to this invention will be described. Materials for the ashes include, as explained above, animals of all kinds inclusive of birds, fishes and shells, parts thereof and their excrements, as well as woods, grasses, plants and grains, their products such as old newspapers and magazines, but exclude radioactive ashes from a nuclear reactor.

For example, seaweeds of all kinds may be dried in the sun to reduce their water content to 30% or less and piled on a bridge for the hearth of a burning oven. A gas burner may then be applied from one side for a complete combustion process for 6 to 12 hours at 725°C-900°C to remove carbon. The ash which drops from the bridge is crashed and sifted, say, by means of a 200-mesh filter. The smaller the particle size of the ashes, the greater the surface area and the ash can emit far infrared radiation more efficiently. Seaweed ash thus collected is in the form of powder with a light grayish color.

As another example, old newspaper and the like may be heated to 900°C - 1500°C. The newspaper ash thus obtained is continually heated in the melted condition at 900°C or over for at least 30 minutes and is suddenly cooled in water to produce a frit. Particles obtained from the frit are colloidal particles. When fired, these particles melt again but there takes place hardly any chemical reaction. When powder of organic ash is fired onto a metal, it is necessary to match the coefficients of expansion of the metal and the ash as closely as possible, say, within 0.7 - 0.9% in the range of 900°C - 1500°C. This problem is solved because the coefficient of expansion of the ashes is increased when they are made into a frit. Particle size of the powder may be further adjusted by means of a bowl mill or the like. If this production method is carried out carefully so as to avoid thermal non-uniformity during a long time of high-temperature thermal decomposition process, the composition obtained as a result will include no non-decomposed salts and there will be sufficient rearrangements of atoms of the substances obtained by decomposition. There will be reduced defects and non-uniformities and the composition and particle sizes will be uniform. Powder thus obtained is dried before being used.

Alternatively, after newspapers and the like are heated to 900°C - 1500°C and melted and further heated for at least 30 minutes, the heating may be stopped to allow the object cool naturally to the normal temperature. The cooled object may then be crashed by means of a bowl mill or the like until particles which can pass through a 200-mesh filter are obtained.

There is no particular limitation imposed on the combustion process, except that the mixing of foreign objects must be carefully prevented. If necessary, foreign objects are removed, and the ash is refined. Ashes may be obtained also by subjecting industrial wastes to a complete combustion process. Compositions of organic ashes thus refined are shown in Table 2 for ashes of seaweeds, Table 3 for ashes of plants, Table 4 for ashes of newspapers, Table 5 for ashes of sardines, Table 6 for ashes of pork, and Table 7 for ashes of defatted soy bean.

**Table 2**

| Element | Content |
|---|---|
| Carbon | Not found |
| Potassium | 12.0% |
| Sodium | 5.83% |
| Magnesium | 4.46% |
| Iron | 5.38% |
| Calcium | 21.4% |
| Chlorine | 14.1% |
| Manganese | 1002ppm |
| Nickel | 470ppm |
| Zinc | 333ppm |
| Copper | 51.6ppm |
| Lead | 5.5ppm |
| Chromium | 2305ppm |
| Silicon | 2.6% |
| Aluminum | 4.68% |
| Lithium | 8.2ppm |
| Iodine | 0.048% |
| Phosphorus | 0.156% |
| Sulphur | 2.43% |
| Strontium | 3070ppm |

**Table 3**

| Element | Content |
|---|---|
| Carbon | Not found |
| Potassium | 54.0% |
| Sodium | 0.32% |
| Magnesium | 2.1% |
| Iron | 0.032% |
| Calcium | 0.88% |
| Chlorine | 2.1% |
| Manganese | 70ppm |
| Nickel | 10ppm |
| Zinc | 0.022% |
| Copper | 11.52% |
| Lead | 5.0ppm |
| Chromium | 100ppm |
| Silicon | 2.5% |
| Aluminum | 3.21% |
| Lithium | 3.2ppm |
| Iodine | 0.3% |
| Phosphorus | 7.68% |
| Sulphur | 2.11% |
| Strontium | 850ppm |

**Table 4**

| Element | Content |
|---|---|
| Carbon | Not found |
| Potassium | 44.0% |
| Sodium | 0.5% |
| Magnesium | 2.1% |
| Iron | 2.3% |
| Calcium | 5.0% |
| Chlorine | 10.2% |
| Manganese | 82ppm |
| Nickel | 10ppm |
| Zinc | 1.2% |
| Copper | 8.1% |
| Lead | 1000ppm |
| Chromium | 105ppm |
| Silicon | 3.2% |
| Aluminum | 2.34% |
| Lithium | 2.8ppm |
| Iodine | 0.03% |
| Phosphorus | 7.68% |
| Sulphur | 2.3% |
| Strontium | 85ppm |

**Table 5**

| Element | Content |
|---|---|
| Carbon | Not found |
| Potassium | 37.0% |
| Sodium | 9.5% |
| Magnesium | 3.7% |
| Iron | 0.23% |
| Calcium | 6.0% |
| Chlorine | 2.8% |
| Manganese | 980ppm |
| Nickel | 100ppm |
| Zinc | 0.13% |
| Copper | 10.0% |
| Lead | 1.3ppm |
| Chromium | 2050ppm |
| Silicon | 1.1% |
| Aluminum | 2.3% |
| Lithium | 5.1ppm |
| Iodine | 0.05% |
| Phosphorus | 14.0% |
| Sulphur | 3.0% |
| Strontium | 2010ppm |

**Table 6**

| Element | Content |
|---|---|
| Carbon | Not found |
| Potassium | 26.0% |
| Sodium | 5.0% |
| Magnesium | 15.1% |
| Iron | 0.51% |
| Calcium | 0.5% |
| Chlorine | 5.2% |
| Manganese | 105ppm |
| Nickel | 70ppm |
| Zinc | 2.3% |
| Copper | 10.5% |
| Lead | 3.1ppm |
| Chromium | 1030ppm |
| Silicon | 1.8% |
| Aluminum | 3.21% |
| Lithium | 1.2ppm |
| Iodine | 0.35% |
| Phosphorus | 10.1% |
| Sulphur | 3.8% |
| Strontium | 53ppm |

**Table 7**

| Element | Content |
|---|---|
| Carbon | Not found |
| Potassium | 23.0% |
| Sodium | 0.4% |
| Magnesium | 3.7% |
| Iron | 0.12% |
| Calcium | 10.0% |
| Chlorine | 11.2% |
| Manganese | 80ppm |
| Nickel | 3.1ppm |
| Zinc | 0.059% |
| Copper | 17.0% |
| Lead | 1.0ppm |
| Chromium | 105ppm |
| Silicon | 2.3% |
| Aluminum | 0.3% |
| Lithium | 1.0ppm |
| Iodine | 0.02% |
| Phosphorus | 6.5% |
| Sulphur | 3.21% |
| Strontium | 203ppm |

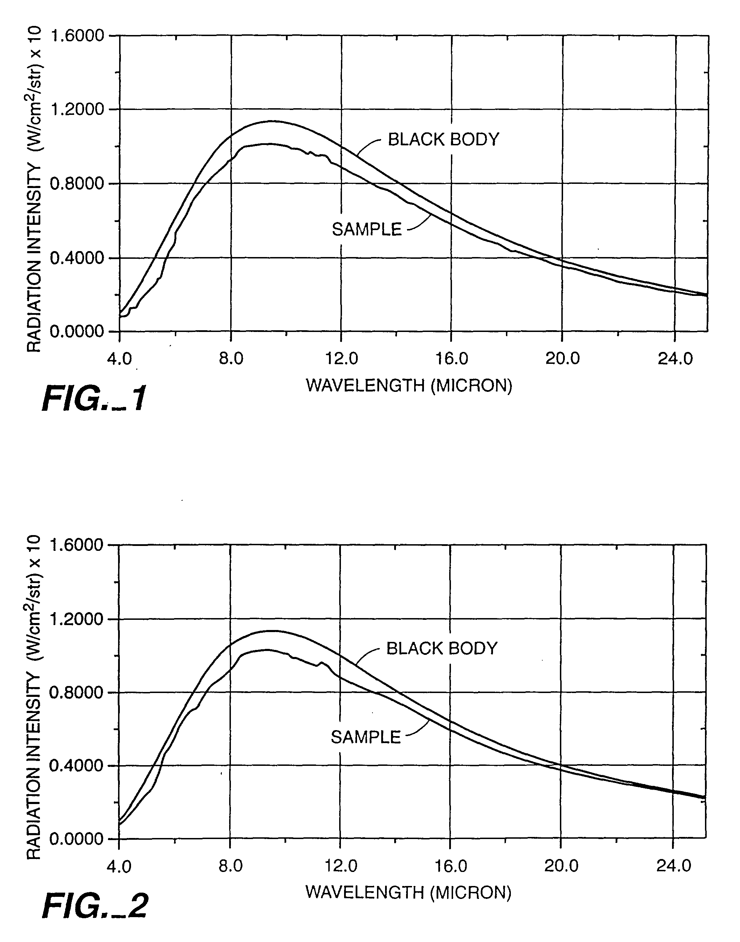

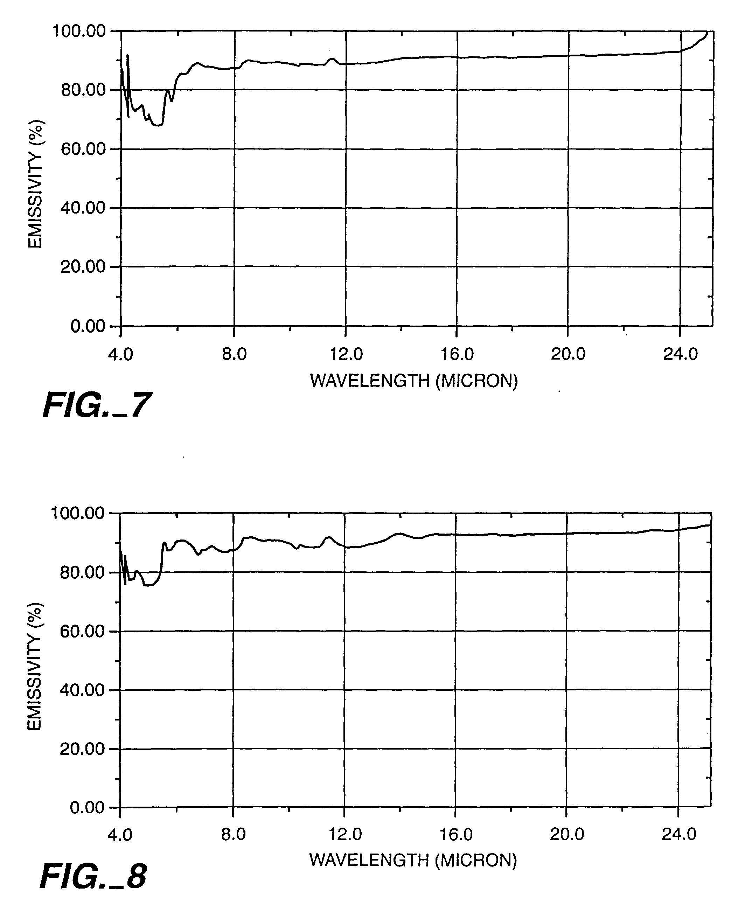

The radiation intensity of these ashes measured at 35°C at the Far Infrared Application Research Association is shown in Figs. 1-6. Use was made of a Fourier transform infrared spectroscope, and radiation spectra were measured in the wavelength range of 4.0 - 24.0µm. The black body radiation intensity shown in Figs. 1-6 represents theoretical values, and the ratios of these measured radiation intensity values to the theoretical black body radiation intensity values are defined as the emissivity and shown in Figs. 7-12.

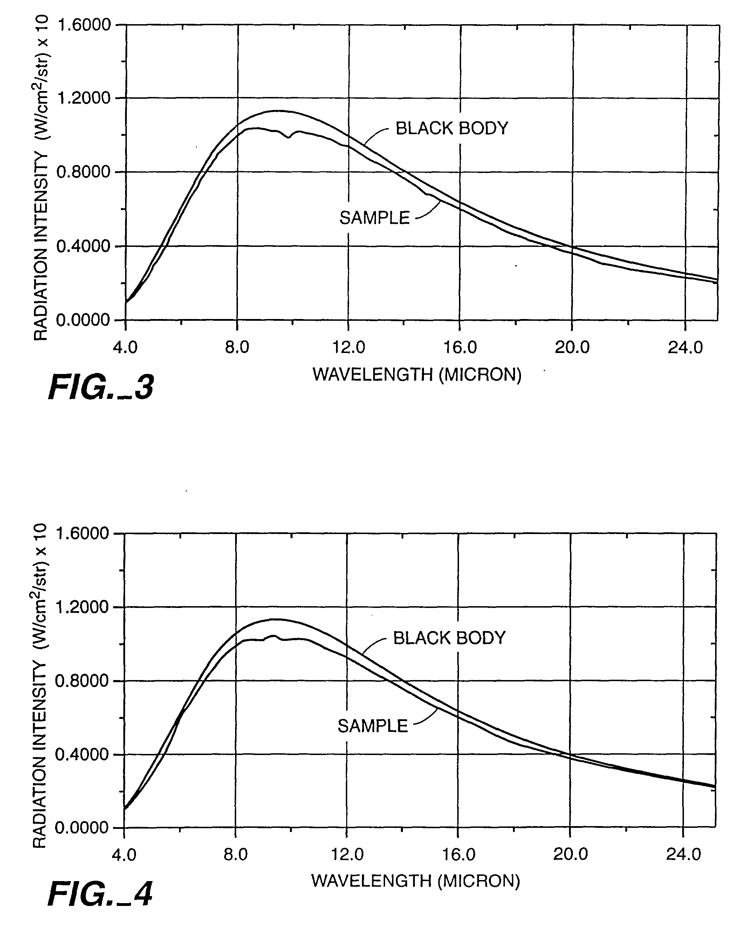

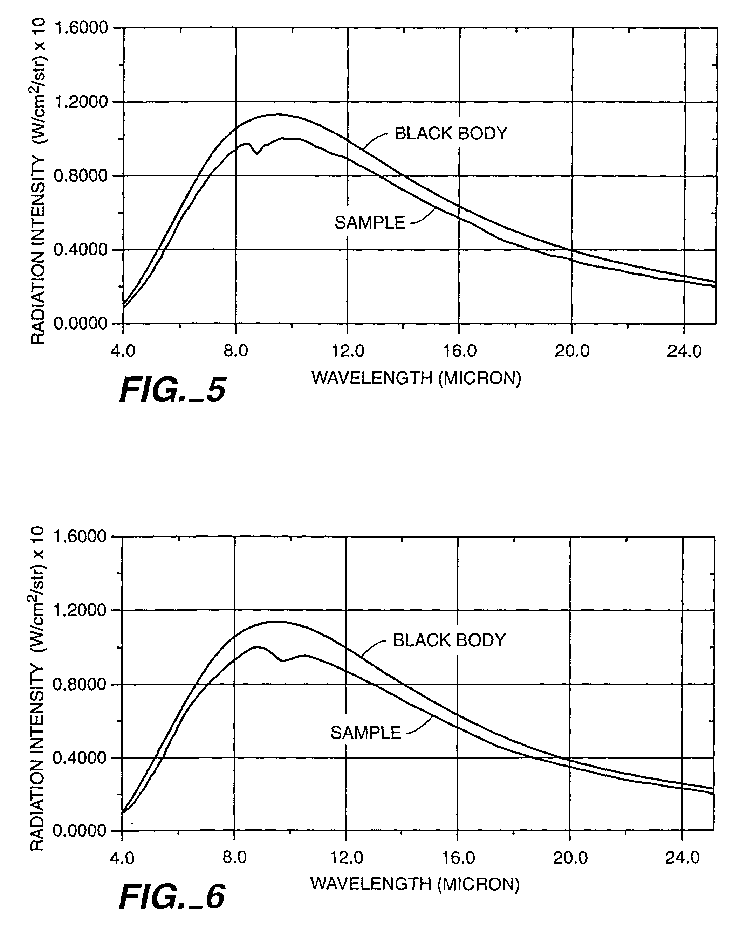

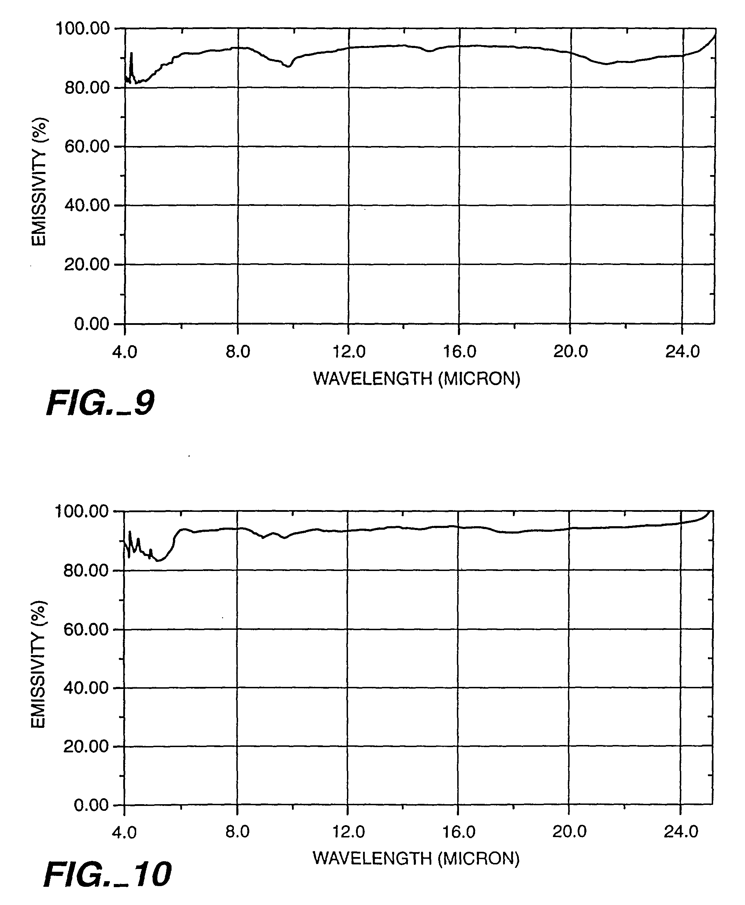

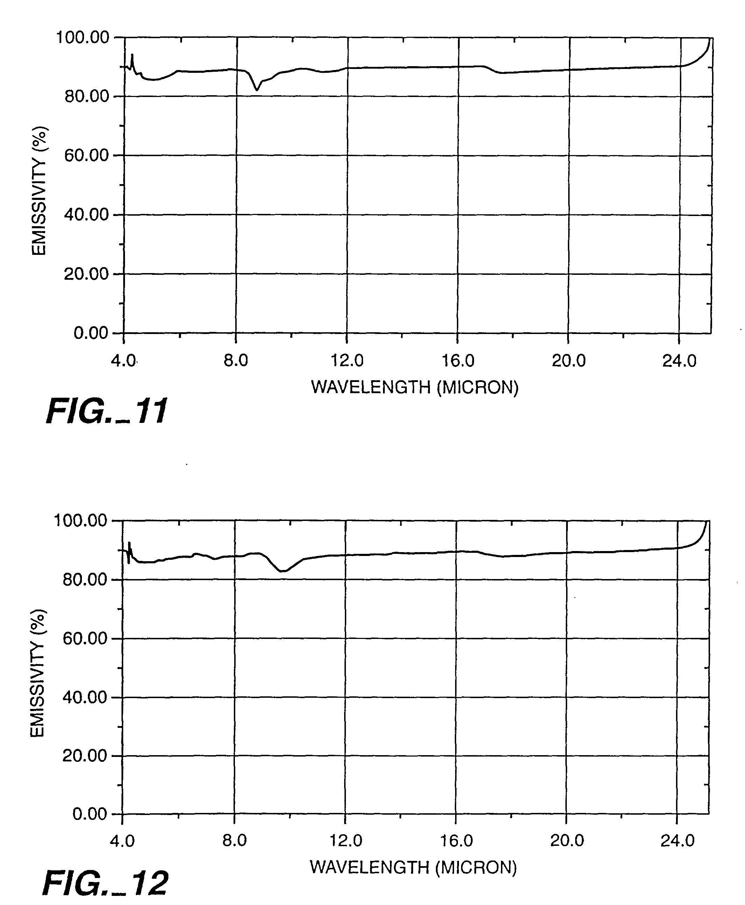

Fig. 1 and 7 are for ashes of seaweeds, Figs. 2 and 8 are for ashes of plants, Figs. 3 and 9 and ashes of newspapers, Figs. 4 and 10 are for ashes of sardines, Figs. 5 and 11 are for ashes of pork, and Figs. 6 and 12 are for ashes of defatted soy beans. These test results show that emissivity at 35°C is very high, being over 80% and about 90% at least in the wavelength range of 8 - 24µm. A peak is present in the emission intensity in the wavelength range of 8-12*µ*m where penetration into human body is known to be high. Organic ashes with emissivity equal to or greater than 80% within the wavelength range of 8-12*µ*m will be used according to this invention.

In what follows, the invention will be described by way of results of some of demonstrative tests, but these examples are not intended to limit the scope of the invention. Throughout herein, percentages in contents are by weight, unless specifically stated otherwise.

### Test No. 1 (Resins)

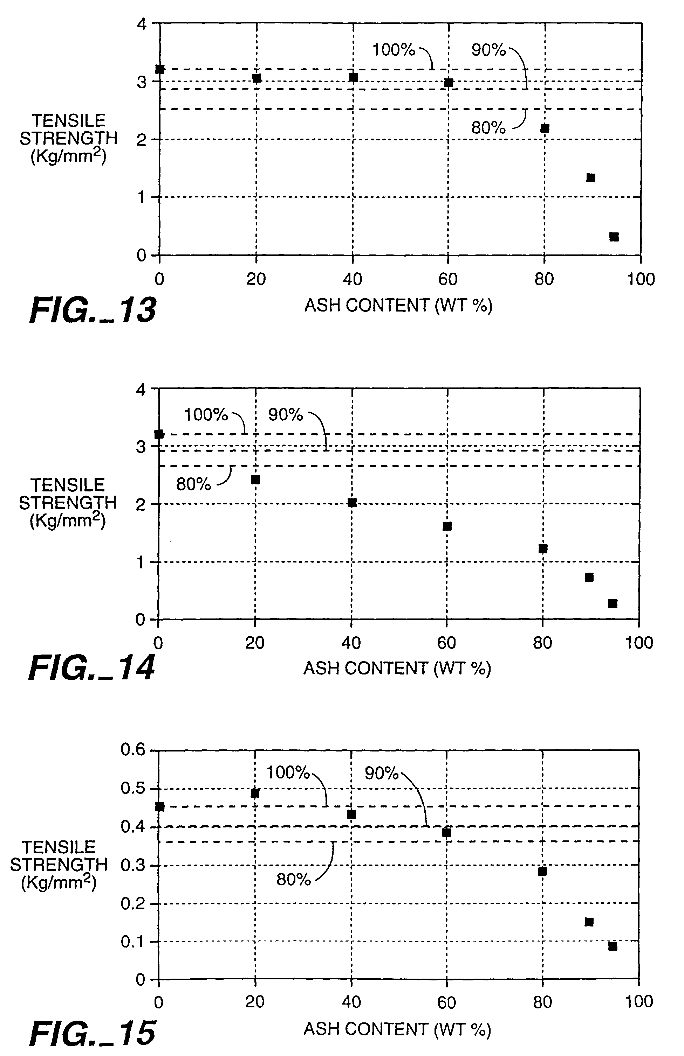

This invention relates to resins containing organic ashes, including polymer resins such as thermoplastic resins, thermosetting resins, foaming styrene and artificial rubbers, as well as natural resins such as pine resin and natural rubbers. If too much ash is contained, however, this can affect the strength of the material adversely. One of representative examples of physical characteristics of resins affected by the mixing of ashes is the tensile strength. In order to investigate this effect quantitatively, test samples were prepared by mixing newspaper ashes at weight ratios of 0%, 20%, 40% 60%, 80% 90% and 95% into high-density polyethylene (HDPE), polypropylene (PP) resin, hard vinyl chloride resin, polyethylene terephthalate (PET) resin and acrylonitrile butadiene styrene (ABS) resin by means of a two-shaft extruder for mixing and kneading. Test pieces were produced from these sample materials according to JIS (Japanese Industrial Standards) K-7113, and their tensile strengths (in units of kg/mm²) were measured by means of a multi-purpose tension tester. The results of these tests are shown in Figs. 13-17. It is thereby learned that the tensile strength of HDPE, hard vinyl chloride resin and ABS resin is nearly constant if the ash content is about 60% or less but it drops rapidly over 80%, dropping to less than one third of the original value and becoming impractical for use if the ash content exceeds 90%. The tensile strength of PP resin drops with the mixing of the ashes, dropping to less than one quarter of the original value if the ash content exceeds 90%. As for PET resin, its tensile strength reaches a maximum when the ash content is 20%. When the ash content is 30%, the tensile strength becomes about the same as when the ash content is zero (or in the no-ash condition). It then begins to drop, becoming less than one quarter of the no-ash condition when the ash content exceeds 90% and having hardly any strength any more. Figs. 13-17 show that the contents of newspaper ashes, at which the tensile strength of a resin drops to 90% and 80% of its value in the no-ash condition, are respectively 65% and 75% for HDPE, 6% and 13% for PP resin, 53% and 63% for hard vinyl chloride resin, 43% and 54% for PET resin, and 62% and 69% for ABS resin.

A method of mixing ashes to ABS resin, as an example of thermoplastic resins, and measuring its radiation intensity will be described next.

Resin particles in the form of pellets or resin powder is mixed with separately prepared newspaper ashes (weight ratio of 2%) in a same container and kneaded together. For this purpose, an apparatus such as a drum blender may be used. During this process, an appropriate coloring agent may be added such that the final product will have a desired color. An extruder of either a single-shaft or two-shaft type may be used to mix and pulverize at about 220°C at which ABC resins melts. Apparatus of many other kinds may be substituted for this purpose as long as they are capable of uniformly dispersing the ashes throughout the resin material.

The grains of ABS resin thus prepared were used to obtain a plastic plate of thickness 2mm by using a press machine at temperature of 200°C. Its radiation intensity was measured by means of a high-sensitivity detector over a wavelength interval of 8-24*µ*m, and the measured intensity values were integrated over the same interval, and the Planck's law was used to obtain the intensity of far infrared radiation emitted per unit area, or in units of W/m². For a plastic plate produced similarly but without newspaper ashes, the intensity value thus obtained was 290. With newspaper ashes mixed in, it increased to 690, indicating that the far infrared radiation intensity is significantly increased if newspaper ashes are mixed. When the mixing ratio of newspaper ashes was reduced to 0.01%, an increase in the intensity was observable in the wavelength interval of 8-24*µ*m. When the ash content was reduced to 0.001%, however, there was only an insignificant increase.

If the mixing ratio of organic ashes is increased, the increase in the effects of far infrared radiation becomes correspondingly significant. If it exceeds 90%, however, the strength of the plastic products drops to such an extent that it may not be possible to keep the intended shape of the product. The preferred mixing ratio is 0.01% to 20%.

When a denture is worn out, ulcers may be formed inside the mouth. It typically takes 5 to 7 days to heal while a correction is made to the denture. If the denture is made of a material containing seaweed ashes by 0.1%, it was discovered that the ulcer can be made to disappear in 2-3 days. Similar effects could be observed by using other kinds of organic ashes.

As another test, cigarette cases were made with natural and polymer resin materials containing organic ashes by 0.01% as an emitter of far infrared radiation. It was ascertained that cigarettes stored in such a case become milder and they lose their stinging sensation to the tongue in a week. When such a resin material was used as a filter, the taste of the cigarette became milder.

### Test No. 2 (Sheets and films)

Inexpensive sheets and films can be produced by using the same resin material containing newspaper ashes as described above by a method such as the extrusion method and the single-shaft and two-shaft drawing methods. Sheets and films functioning as an emitter of far infrared radiation can also be produced by applying or coating such newspaper ashes on surfaces of an ordinary sheet or film not containing any organic ashes. It does not matter whether such application or coating process is carried out before or after the sheet or film is drawn.

Such radiation emitting sheets and films may be used as a single sheet or film, but they may also be used in layers for the purpose of providing additional advantages. In the case of a multi-layered sheet of film, a highly effective product can be obtained if one or more of the plurality of layers contain organic ashes.

A sample plate of ABS resin with thickness 2mm containing newspaper ashes and a comparison plate of ABS resin with thickness 2mm not containing any ashes were each subjected to a single-shaft drawing process at 200°C to produce thin sheets of thickness 0.5mm and their far infrared radiation intensity values were measured similarly as described above, corresponding to electromagnetic radiation of 8-24*µ*m. There was no change in the measured values, indicating that the radiation emitting property is not affected by the drawing process. Similar results were obtained with organic ashes of other kinds.

### Test No. 3 (Coating materials)

This invention also relates to coating materials containing organic ashes by 0.01% - 90% with respect to the base coating material (defined as the material before diluted by a solvent such as a thinner, toluene and water to make what is commonly referred to as a coating material). If the ash content is 0.01%, effects of adding far infrared emission property is already significant.

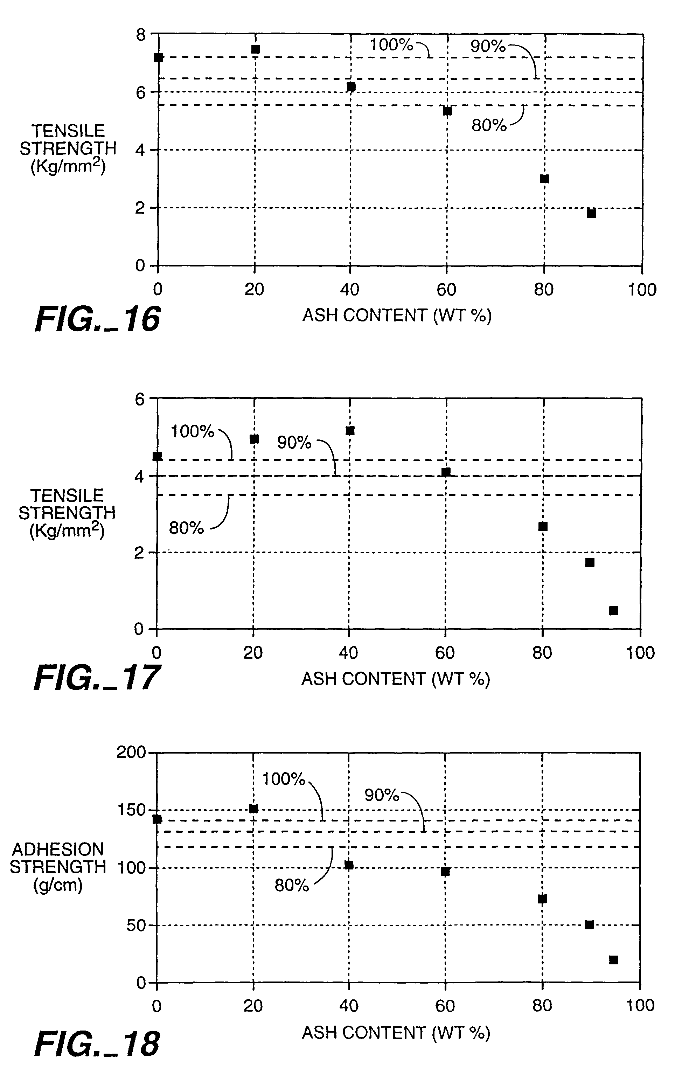

One of representative physical characteristics of coating materials affected by the mixing of newspaper ashes is the adhesive strength. If the adhesive strength of a coating material is too low, it begins to peel off easily and ceases to be useful as a coating material. In order to investigate the effects of the mixing ratio of newspaper ashes on the adhesive strength, newspaper ashes were mixed into coating materials of vinyl chloride resin (with molecular weight of 50000), aminoalkyd resin, unsaturated polyester resin and acryl lacquer by 0%, 20%, 40%, 60%, 80%, 90% and 95%. Adhesive strengths were measured (in units of g/cm²) by the so-called adherometer method using an ivory knife of width 4mm to peel off the coating material from the coated surface and to thereby measure the resistance on the knife. The results of the tests are shown in Figs. 18-21.

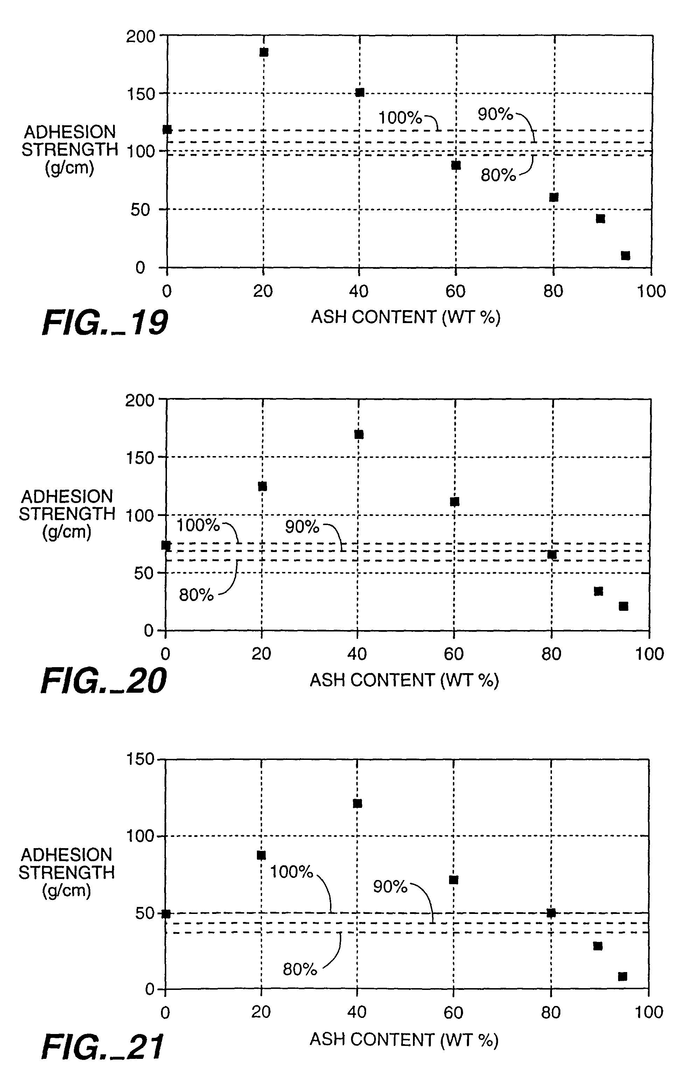

With the sample of vinyl chloride resin, Fig. 18 shows that the adhesion strength attains its maximum value when the ash content is about 20%, slowly decreasing if the ash content is further increased, dropping rapidly if the ash content is greater than 80% and becoming about one tenth of the original value if the ash content much exceeds 90%. The contents of newspaper ashes, at which the adhesion strength of a coating material with the vinyl chloride sample became 90% and 80% of that of an ash-free sample, were respectively 33% and 37%. With the aminoalkyd resin sample, Fig. 19 shows that the adhesion strength attains a much greater maximum value when the ash content is 20% than in the control condition, dropping gradually thereafter and dropping rapidly and becoming about one fifth of the maximum value if the ash content exceeds 90%. The contents of newspaper ashes, at which the adhesion strength of a coating material with aminoalkyd resin sample became 90% and 80% of that of an ash-free sample, were respectively 53% and 58%. With unsaturated polyester resin sample, Fig. 20 shows that the adhesion strength reaches its much greater maximum value when the ash content is 40% than in the control condition, dropping gradually thereafter and dropping to less than one fifth of the maximum value if the ash content exceeds 90%. The contents of newspaper ashes, at which the adhesion strength of a coating material with unsaturated polyester resin sample became 90% and 80% of that of an ash-free sample, were respectively 79% and 82%. With the acryl lacquer sample, Fig. 21 shows that the adhesion strength reaches its much greater than maximum value when the ash content is 40%, dropping gradually thereafter and becoming about one fifth of the maximum value when the ash content exceeds 90%. The contents of newspaper ashes, at which the adhesion strength of a coating material with acryl lacquer sample became 90% and 80% of that of an ash-free sample, were respectively 83% and 86%.

For emulsions serving as a coating material, the maximum allowable content of newspaper ashes changes significantly, depending on the condition of the surface to be coated. When a flat smooth coated surface is required, ashes may be mixed in up to a critical pigment volume concentration which is uniquely determined by a relationship among the pigment, newspaper ashes and the binder. In the case of an acryl emulsion serving as a coating material, the critical pigment volume concentration was 75% if the binder was acryl latex. In other words, newspaper ashes could be mixed by up to 75% by weight to obtain a smooth coated surface. If a flat finish is desired, however, more newspaper ashes could be added by up to 95% without adversely affecting stability of the ashes. It was similar with vinyl acetate emulsions, except the critical pigment volume concentration in the case of newspaper ashes was 71%.

Coating materials can be applied almost to any product and on any area. Thus, if newspaper ashes are mixed to an oil-based or water-based coating material, say, by 10%, the property of emitting far infrared radiation can be provided to almost any existing item. If charcoal is mixed, a coating material thus prepared can be used only for making anything black because charcoal itself is very black. Newspaper ashes, by contrast, can be mixed into coating materials of any color. Organic ashes may be mixed also into a dye by 0.01% to 30%.

Examples of coating material into which organic ashes can be mixed include oil-based coating materials, water-based coating materials, other solvent-based coating materials and powder materials. In other words, boiling oil, mixed paints, oil varnish, anti-rust paints, roof paints, oil-based enamels, aluminum paints, lacquers of many kinds, phenol resin coating materials, alkyd resin coating materials, synthetic resin mixed paints, synthetic resin anti-rust paints, thermosetting aminoalkyd resin coating materials, acid setting aminoalkyd resin coating materials, thermosetting acryl resin coating materials, polyester resin coating materials, polyurethane resin coating materials, epoxy resin coating materials, etching primers, vinyl chloride resins, rubber chloride coating materials, tar epoxy resin coating materials, acryl emulsion coating materials, and aqueous varnish are all included.

Organic ashes can be mixed into such coating materials at any stage between the time of production of the coating material and the time of actual coating. During the production process, organic ashes may be preliminarily mixed with a coloring agent at an appropriate rate. The ashes may be preliminarily mixed in a solvent or other material. It goes without saying that ashes may be mixed with more than one of these components.

If ashes are to be mixed at the time of actual coating, they may be added either directly or by using an appropriate amount of a dispersant or the like and stirring to disperse them. If a device such as an air spray is used for the coating, a mechanism may be provided for mixing the base material with ashes and dispersing them.

It is to be noted that, since organic ashes do not participate in chemical reactions and are thermally stable at temperatures up to 700°C, they can be mixed not only into coating materials for use only at normal temperatures but also coating materials intended to be subjected to a firing process.

A vinyl chloride resin powder coating material can be produced by preliminarily adding 2% of newspaper ashes to the resin before the melting and kneading process by a melt-blend method and the usual steps of melting, kneading, cooling, pulverizing and classifying, after coloring agents, additives and hardening agents are preliminarily mixed in. A 10cm square test iron plate was coated with the material thus obtained with firing. For comparison, a similar coating material was prepared without mixing any ashes in and another test iron plate was similarly coated with this comparison material with firing. The far infrared radiation intensity values of these two test iron plates were measured in units of W/m² at 35°C corresponding to electromagnetic radiation of wavelengths in the range of 8-24µm. It was 255 with the coating material without ashes but it was 676 for the coating material containing newspaper ashes. This again shows that the mixing of newspaper ashes significantly increases the intensity of far infrared radiation.

As another test, a room was painted with a paint containing ashes from plants by 3 weight %. Insomnia of a patient was cured after the patient slept in this room for 10 days. Another patient had a chilly constitution, but it improved after about 14 days. Next, the room was coated with a paint containing ashes from plants by 30 weight %. Under this condition, insomnia was cured in 2 days and a chilly constitution was improved in about 2 days. Similar results were obtained by using paints containing organic ashes of other origins.

### Test No. 4 (Construction materials)

The invention also relates to construction materials containing organic ashes by 0.01% to 30%.

Examples of construction materials include thermal insulators, wall papers, natural and polymer resins such as nylon and vinyl and cloth materials for covering walls and ceilings, cements, blocks, roof tiles, bricks, mortars and plates. If the ash content is less than 0.01%, no intended effects could be obtained. From the point of view of strength, it is preferred that the mixing ratio be less than 30%. To produce construction materials according to this invention, ashes from plants were mixed with a water-based coating material at a rate of 1 weight %, stirred, dispersed uniformly and spray-coated on commercially available construction materials. As shown in Table 8, far infrared radiation intensity values were measured in units of W/m² for each of these construction materials without any coating (first column), with a coating material containing ashes from plants by 1% (second column), with a coating material containing wood charcoal by 1% (third column) and with a coating material containing wood charcoal by only 0.002% such that the resulting color will be about the same as when the coating material with ashes from plants is used. Table 8 shows that the coating materials containing ashes of plants by 1% have much greater emission intensity of far infrared radiation than when there is no coating and the effects are greater than when the coating material contains wood charcoal at the same rate (1%). If the content of wood charcoal is reduced such that the resulting color would be about the same as when the coating material containing organic ashes is used, the charcoal content becomes as small as 0.002% and the far infrared radiation intensity values are not significantly larger than when no coating material was used, the differences being within the limit of error of measurement.

**Table 8**

| Construction | | Radiation intensity values | | |
|---|---|---|---|---|
| Material | No Coating | Coating material with ashes (1%) | Coating material with wood charcoal (1%) | Coating material with wood charcoal (0.002%) |
| Gypsum board | 305 | 735 | 638 | 315 |
| Wallpaper | 287 | 732 | 623 | 290 |
| Cloth materials | 290 | 729 | 630 | 293 |
| Cement | 310 | 735 | 622 | 306 |
| Block | 310 | 733 | 630 | 314 |
| Roof tile | 370 | 733 | 632 | 373 |
| Brick | 364 | 728 | 615 | 361 |
| Mortar | 332 | 731 | 620 | 335 |
| Plywood | 292 | 725 | 615 | 290 |

Similar results could be obtained if organic ashes were directly mixed with the construction materials by properly controlling the amount of the ashes. Construction materials thus produced have superior strength against washing. Similar effects were observed with organic ashes of different kinds.

A patient was made to lie on constructional cloth materials (including synthetic fiber materials comprising polymer resins such as nylon, polyesters and vinyls, as well as natural fibers such hemp, cotton and wool) containing newspaper ashes by 3%, and his systolic pressure dropped by 30mmHg from 185mmHg to 155mmHg in 15 minutes. In the meantime, the diastolic pressure dropped from 98mmHg to 83mmHg. In 8 hours, improvements were observed in palpitation, breathlessness, stiff shoulders and pains in the waist.

### Test No. 5 (Ointments)

The invention also relates to ointments containing organic ashes by 0.01% to 30%. If the content of ashes was less than 0.01%, no intended effects could be obtained. If the content exceeds 30%, the oil component of the ointment becomes separated and the product could not be used as an ointment.

As a test, seaweed ashes were mixed into commercially available ointments including Jubela ointment (trade name of Eisai, Inc.), Oronine H ointment (trade name of Otsuka Pharmaceutical Factory, Inc.) and Mentholatum cream (trade name of Roht Pharmaceutical Co., Ltd.) at rates of 0.01% to 30% and uniformly kneaded by using a mixing apparatus. The kneaded ointments are gray in color but their gray color substantially disappears when they are applied in a thin film on the skin and rubbed in. Similar effects could be obtained with organic ashes from other sources.

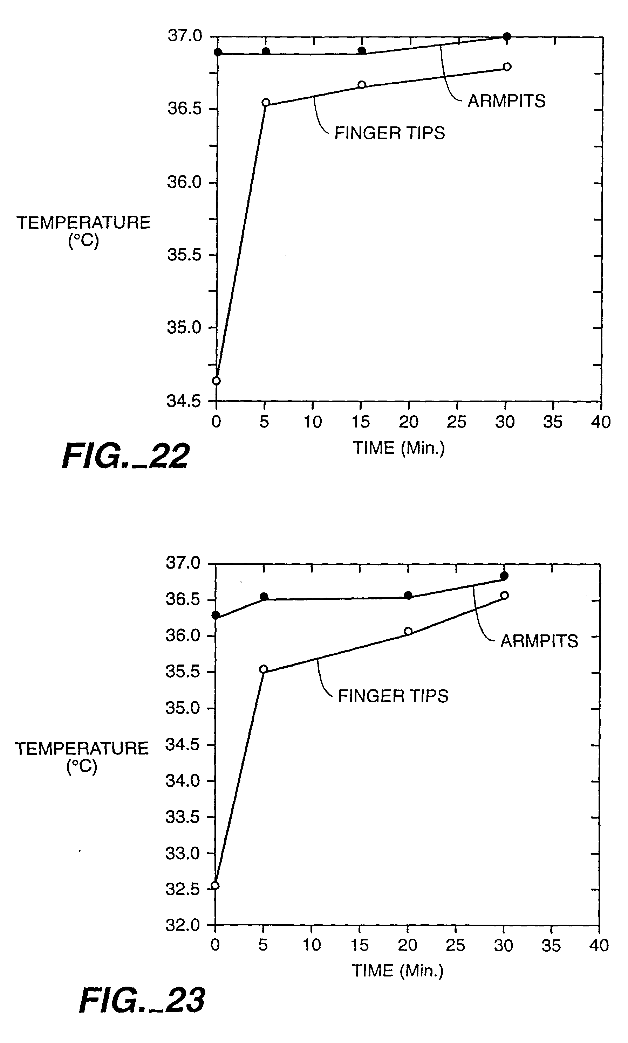

For studying their effects on body temperatures, a sample ointment was prepared, containing (per 1g of the ointment) 20mg of tocopherol, 5mg of vitamin-A oil (5000 units as vitamin A), 20mg of seaweed ashes and additives including isopropyl myristicate, propylene glycol, polysolvate 60, lanolin alcohol, cetanol, methylparaben, propylparaben, and sodium edetate. A 50-year old female patient with system lupus erythematosus (which is an autoimmune disease) and allergic rhinitis was made to rest quietly for 30 minutes inside a room maintained at 25°C. Fig. 22 shows the change in body temperature of this patient. When the ointment described above was applied (at time = 0 ) to a frontal left-hand side of the head of the patient, her body temperature was 36.9°C in the pit of the right arm and her fingertip temperature (measured with the thermometer held between the thumb and the index finger) was 34.5°C. When measurements were taken 5 minutes later, the armpit body temperature was 36.9°C and the fingertip temperature was 36.5°C After 30 minutes, they were respectively 37.0°C and 36.7°C, increasing by 0.1°C and about 2.2°C. The fact that the fingertip temperature on the right-hand side increased although the ointment was applied on the frontal left-hand side of the head implies that the blood circulation improved throughout the entire body. Moreover, the patient's allergic rhinitis improved significantly, and so was the congestion in the eyes due to the systemic lupus erythematosus. This seems to imply that there is an immunization center near the left frontal lobe and an allergic condition can be improved by acting on this center. Similar effects were observed with organic ashes of different kinds.

### Test No. 6 (Cataplasm poultices)

The invention also relates to cataplasm poultices containing organic ashes by 0.01% to 20%.

As examples, medicaments were prepared by mixing organic ashes by 0.01% to 20% into commercially available cataplasm poultices such as Salonpas (trade name of Hisamitu Pharmaceutical Factory, Inc.) and Tokuhon (trade name of Tokuhon, Inc.), kneading and applying the mixture to a base of a paper or cloth material. If the ash content is less than 0.01%, no desired effects can be observed. If the content exceeds 20%, on the other hand, the adhesive characteristic of the cataplasm poultice is adversely affected.

For studying the effects on body temperatures, a sample cataplasm poultice was prepared, containing (per 1m³) 10.5g of glycol salicylate, 4.2g of L-menthol, 4.2g of acetic acid extract of Vitamin E, 0.21g of glycyrrhizic acid, 0.945g of mustard extract, 0.41g of newspaper ashes, and additives inclusive of dibutyl hydroxytoluene, rosin ester and perfume. A 65-year old female patient with a history of myocardial infarction was made to rest for 30 minutes quietly inside a room kept at 25°C, and the cataplasm poultice described above was attached to the wrist of her left hand (at time = 0). The subsequent changes in the patient's body temperature are shown in Fig. 23. At first, the patient's body temperature was 36.2°C in the pit of the right arm and the fingertip temperature (measured as above) was 32.5°C. When measurements were taken 5 minutes later, the armpit temperature was 36.5°C and the fingertip temperature was 35.5°C After 30 minutes, they were respectively 36.7°C and 36.5°C, increasing by 0.5°C and about 3.2°C, respectively. The fact that the fingertip temperature on the right-hand side increased although the cataplasm poultice was applied to the wrist of the left hand implies that the blood circulation improved throughout the entire body. This indicates a possibility of improving the patient's congelation and hypothermia. As for the content of newspaper ashes, a concentration in the range of 0.05% to 5% is preferred.

Next, brain waves were measured on eight test subjects consisting of a 20-year old female, a 28-year old male, a 38-year old female, a 30-year old male, a 47-year old female, a 58-year old female and a 37-year old female. None of these test subjects had any particular history of diseases.

The alpha-wave is a kind of brain wave said to be emitted when a person is in a relaxed, peaceful frame of mind. The rate of alpha-wave emission was 6.1% when a simple piece of cloth used for cataplasm poultice not containing any medicament was attached to the body, 7.3% when nothing was attached (as a control), 7.5% when use was made of a commercially available ointment, 9.5% when a commercially available cataplasm poultice was attached, 15.0% when use was made of the ointment of Test No. 5 containing seaweed ashes, and over 16.6% when the cataplasm poultice described above containing newspaper ashes was used.

Because cataplasm poultices and ointments according to this invention contain natural far infrared radiation emitters (seaweed ashes, in particular), test subjects could relax due to improved blood circulation and recovery from fatigue, and hence emitted more alpha-waves. The cataplasm poultices were more effective than the ointments for the emission of alpha-waves. This was probably due to the localized attachment at pressure of 0.13 atmosphere. Similar effects could be observed with organic ashes of other kinds.

### Test No. 7 (Adhesive tapes)

The invention also relates to adhesive tapes with its base material or adhesive material containing organic ashes by 0.01% to 20%. If the ash content is less than 0.01%, no desired effects can be obtained. If the content exceeds 20%, the strength of the base material and the adhesive characteristic of the adhesive material become insufficient to the extent that the product can no longer function adequately as an adhesive tape.

As an example, an adhesive tape was produced by mixing 0.1g of ashes of defatted soy beans to a medicament per 10cm² of surface area. A 27-year old male having a pain in the first molar on the upper left-hand side of the jaws with acute purulent pulpitis was treated by cutting this tape into squares of sides 0.5cm and pasting them at the position of the pain (the upper left-hand side of the jaws) and one of the so-called vital spots, according to the oriental medical science, located at the junction of the roots of the thumb and the index finger. The pain disappeared substantially entirely in 30 minutes.

This healing process is probably based on the principle of acupuncture and moxibustion, and the result hereby obtained was similar to what can be accomplished by applying gold particles at the location of pain and silver particles at this vital spot, (the gold and silver particles being products of Sakamura Lab & Co., sold under the trade name of Magrain, Permission No. 48B-114 for treating stiffness in the neck, shoulder and waist, as well as pains in the neck and shoulder derived from the stiffness.) The effect is probably due to the electrical potential difference between two different kinds of metal which accelerates the blood circulation in a body. In another test, an adhesive tape according to this invention was attached to the position of pain at the waist of a patient. The pain disappeared in three days. Similar effects were observed with organic ashes of different origins.

### Test No. 8 (Accessories)

This invention also relates broadly to all kinds of a product having attached to its surface a material such as metallic and coating materials containing organic ashes of plants by 0.01% to 50% either by firing a metallic (magnetic or otherwise) or resin material or by means of an adhesive. The invention also relates to such products using an adhesive with such an ash content. If the ash content is less than 0.01% in all these examples, no desirable effects are observed. From the point of view of strength, the preferred ash content is 30%.

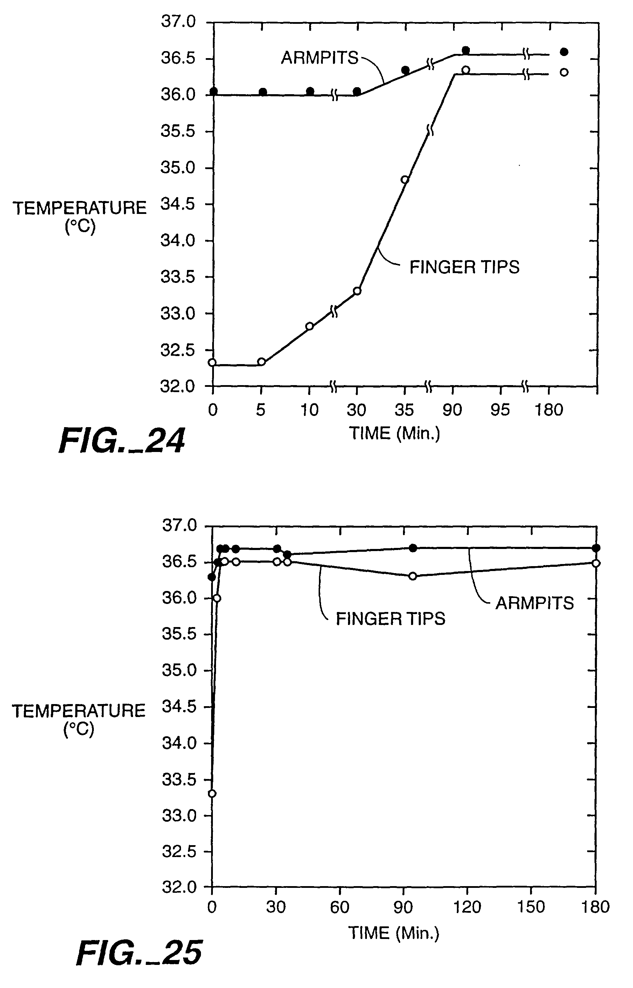

As an example, a ring was produced by firing to attach a coating material containing plant ashes by 10% onto the surface of a metal alloy containing zinc by 2%, gold by 12%, silver by 13%, palladium by 70% and copper 3%. After a 20-year old female patient suffering from insomnia was made to rest quietly for 30 minutes inside a room kept at 25°C, the ring described above was put on the ring finger of the patient's left hand (at time = 0). The subsequent changes in the body temperatures of the patient are shown in Fig. 24. The patient's body temperature was initially 36.0°C in the right armpit, and the fingertip temperature (measured as described above) was 32.3°C. When temperature measurements were repeated 5 minutes later, the armpit temperature was 36.0°C and the fingertip temperature was 32.3°C, showing no change. After 30 minutes, they were respectively 36.0°C, showing still no change, and 33.3°C, showing a slight increase of about 1.0°C. After 60 minutes, they were 36.5°C and 34.5°C, both showing an increase. Still 60 minutes later, they were 36.5°C and 36.1°C, showing stabilization. Thereafter, the patient kept the ring on, and the insomnia improved in two days and was completely gone in ten days. Similar results were obtained with a ring having a hole filled with a resin material containing plant ashes by 10%. Such results were also obtained with organic ashes of other kinds.

Similar results are observable with patients who put on an accessory such as a glass or ceramic necklace or bracelet produced by dissolving and molding or firing a material containing sardine ashes by 10% in the material or in the additive. Similar results were obtained when organic ashes were added to the glaze. As an example, a bracelet was produced by firing a frit made from sardine ashes onto a metallic alloy of zinc 30%, silver 45% and gold 25%. After a 33-year old male patient with stiffness in shoulders was made to rest for 30 minutes quietly inside a room kept at 25°C, the bracelet was put on the wrist of the patient's left hand (at time = 0). The patient's body temperature was initially 36.5°C in the right armpit, and the fingertip temperature (measured as described above) was 35.5°C. When the temperature measurements were repeated 7 minutes later, the armpit temperature was 36.5°C and the fingertip temperature was 36.4°C. Blood pressure of the patient was measured throughout the test. Initially, the patient's systolic pressure was 148mmHg, diastolic pressure was 90mmHg, and the pulse rate was 66 times/minute. Seven minutes later, the systolic pressure was 133mmHg, diastolic pressure was 80mmHg, and the pulse rate was 66 times/minutes. In the meantime, the pressure rate product.dropped from 9768mmHg/min to 8778mmHg/min and the total peripheral resistance (resistance when blood passes through a peripheral vessel) dropped from 1590dyne/sec/cm⁻⁵ to 1480dyne/sec/cm⁻⁵. This seems to indicate that seaweed ash has dilatory effects on peripheral blood vessels.

This test indicates that desired effects show more slowly than when an ointment or a cataplasm poultice of this invention is used, but that the effects are undeniably present. Since far infrared radiating substances, and seaweed ashes in particular, are believed to have the property of accelerating absorption of many kinds of minerals such as zinc ions contained in the bracelet, it is preferred that such an accessory be used over a long period of time.

### Test No. 9 (Medical products)

This invention relates also to medical products including a paper material which contains organic ashes by 0.01% to 30% or a material such as resin film containing organic ashes by 0.01% to 80%. If the ash content is less than 0.01%, no desirable effects are obtained. From the point of view of the strength of the product, the ash content is preferably 30% or less.

As an example, paper and resin sheets containing ashes of pork by 10% and emitting far infrared radiation were produced. It was observed that the systolic pressure of a patient who lay on it dropped by 10mmHg from 174mmHg to 164mmHg and his diastolic pressure from 85mmHg to 73mmHg in seven minutes. If a patient not only lies on such a sheet but also covers himself with another so as to be sandwiched therebetween both from above and from below, it takes only four minutes to obtain the same results. Similar results were obtained by using organic ashes of different kinds. It is medically very important in emergency situations, for example, in the case of a cerebral hemorrhage that the blood pressure of a patient can be lowed without administration of any medicine.

At present in Japan, paramedics sent to emergency situations are ordinarily not permitted by law to inject medicine to a patient to lower the patient's blood pressure or to dilate blood vessels. In the case of an ischemic heart disease and, in particular, in the case of angina pectoris or acute myocardial infarction, the probability of dilating the patient's blood vessels and causing the blood to return to the coronary artery will increase if a far infrared radiating sheet mixed with organic ashes according to this invention is used by the emergency crew to wrap up the patient and if such ashes are mixed in the cloth part of the stretcher.

Cerebral hemorrhage takes place where the systolic pressure exceeds the inner pressure of the brain. In order words, the higher the blood pressure, the greater the hemorrhage and the more severe the damage, increasing the possibility of resulting in a permanent damage or even death. If a paper sheet mixed with organic ashes is used to cover the pillow for an emergency patient, if a far infrared radiating sheet is used as the surface material of a pillow, or if organic ashes are mixed with the stuffing of a pillow, its user can obtain the benefit of far infrared radiation through such a pillow. Since a pillow is used frequently in medical emergency situations for keeping the head of the patient at an elevated position, and since the pillow touches the patient's head and neck directly, far infrared radiation through a pillow can be very effective. Such pillows are not only effective in an emergency medical care, but also usable beneficially in an ordinary medical care as well as in everyday life situations. It may be useful to equip ambulance cars with arm rings and/or leg rings made of a metal or resin material mixed with organic ashes so that they can be put on a patient while the patient is being transported.

Next, calcium antagonists with vasodilatory property will be considered. Examples of commercially available calcium antagonist with vasodilatory property include Hypoca (trade name: Yamanouchi Pharmaceutical Co., Ltd.), Coniel (trade name: Kyowa Hakko Kogyo Co., Ltd.), Nivadil (trade name: Fujisawa Pharmaceutical Co., Ltd.) and Rocornal (trade name: Mochida Pharmaceutical Co., Ltd.) for hypertension, angina pectoris, renoparenchymal hypertension, after-effects of cerebral infarction and/or after-effects of ventricular hemorrhage. Most of these are orally administered and their concentration in blood reaches a maximum value in 1 - 2 hours after its rise. In other words, these commercially available Ca antagonists take too long in lowering the blood pressure to be useful in the case of an emergency medical care. In tests for light and medium cases of hypertension, effectiveness (defined as the fraction of persons for whom a medicament is found to be effective) is usually about 84.2%, and it is about 87.5% in serious hypertension cases. It is also well known that these medicaments can lower the systolic pressure significantly but the diastolic pressure cannot be lowered to a significant degree but only by a few mmHg.

With sheets according to this invention, containing pork ashes and having the property of emitting far infrared radiation, blood pressure can be lowered in a short time, and the effectiveness was nearly 100%. This makes them extremely useful in emergency situations. Moreover, although the prior art Ca antagonists mentioned above are known to have side effects such as an increase in glutamic oxaloacetic transaminase (GOT) and glutamic pyruvic transaminase (GPT) in the liver and an increase in uric acid, blood urea nitrogen (BUN) and creatinine in the kidney, sheets according to this invention containing pork ashes have no side effects and are very safe.

At the time of discharge from the body, the concentration of these prior art Ca antagonists in the blood decreases only very slowly. It becomes about one half of its peak value after 6 hours, and a complete discharge takes about 12 hours. This means that the danger of side effects remains for a long period of time. When a sheet containing pork ashes according to this invention is removed from a patient, by contrast, the lowering of the blood pressure and the rise in the peripheral body temperature due to the far infrared radiation stop within 3-5 minutes.

It has also been discovered that graying hair gradually regains its original black color in about 8 months if a hair lotion containing seaweed ashes by 3% is used. If use is made of a lotion containing seaweed ashes by 20%, it was discovered that graying hair begins to turn black in about 1.5 months. This is probably because far infrared radiation, like a massage, promotes blood circulation.

### Test No. 10 (Health items)

After a 38-year old female patient with the history of constantly using a therapeutic magnetic ball attached to an adhesive tape (sold under the trade name of Elekiban by Pip Fujimoto Co.) was made to rest for.30 minutes quietly inside a room kept at 25°C, the tape described in Test No. 3 having seaweed ashes mixed with an adhesive was placed at four shoulder positions on her body and the tapes with the therapeutic magnetic ball were attached thereover (at time = 0). The subsequent changes in the patient's body temperatures are shown in Fig. 25. The patient's body temperature was initially 36.3°C in the right armpit, and the fingertip temperature (measured as explained above) was 33.3°C. Immediately thereafter, the body temperature began to rise until the temperature in the right armpit reached 36.5°C and the fingertip temperature reached 36.0°C. Four minutes after the start, they were respectively 36.7°C and 36.5°C. They remained stabilized thereafter at 5 minutes, 10 minutes and 30 minutes. The armpit temperature dropped to 36.6°C 35 minutes after the start. The armpit temperature reached 36.7°C and the fingertip temperature reached 36.5°C 95 minutes after the start, remaining stabilized thereafter. The patient said that both her hands and feet felt warmer than when only the therapeutic tape with the magnetic ball was used.

### Test No. 11 (Paper products)

The invention also relates to paper products containing organic ashes by 0.01% to 30%. If the ash content is less than 0.01%, no desirable effects are obtained. Ash contents over 30% are not preferred because strength of the paper product is thereby adversely affected.

As an example, paper sheets capable of emitting far infrared radiation were prepared by mixing seaweed ashes by 5% into the pulp for the production of the paper or into the additive to be coated thereon or absorbed thereby. Products made from such paper sheets are useful not only in hospitals but also at homes for their ability to emit far infrared radiation. If cups and plates are produced with such paper sheets, food can be kept warm for a longer period of time. Face masks made with such paper sheets are advantageous because air is warmed before it is breathed in. If such paper sheets are used cosmetically, they serve to gradually remove stains and freckles from the skin. Cardboard boxes made with paper sheets according to this invention can keep vegetables, plants, flowers and fruits fresh as they are shipped. Still better results are obtained if an aluminum sheet is attached inside. Similar effects can be obtained by mixing organic ashes of other kinds.

When a paper product containing plant ashes by 0.01% was used for packaging cigarettes, the cigarettes kept inside became milder in taste and the stinging sensation typical of cigarettes disappeared in three weeks. When the ash content was increased to 25%, the change in the taste became more pronounced, the cigarettes becoming mild in about 12 hours and the stinging sensation disappearing within a day.

As another example, caps were made with paper and cloth materials having ashes of plants either mixed therewith or attached thereon by 1%. Graying hair of a person wearing such a cap gradually turns black in ten months. If the ash content is increased to 25%, the color change takes place in about three months. This is probably due to improved blood circulation. Similar effects were observed with organic ashes from different sources.

### Test No. 12 (Cloth materials)

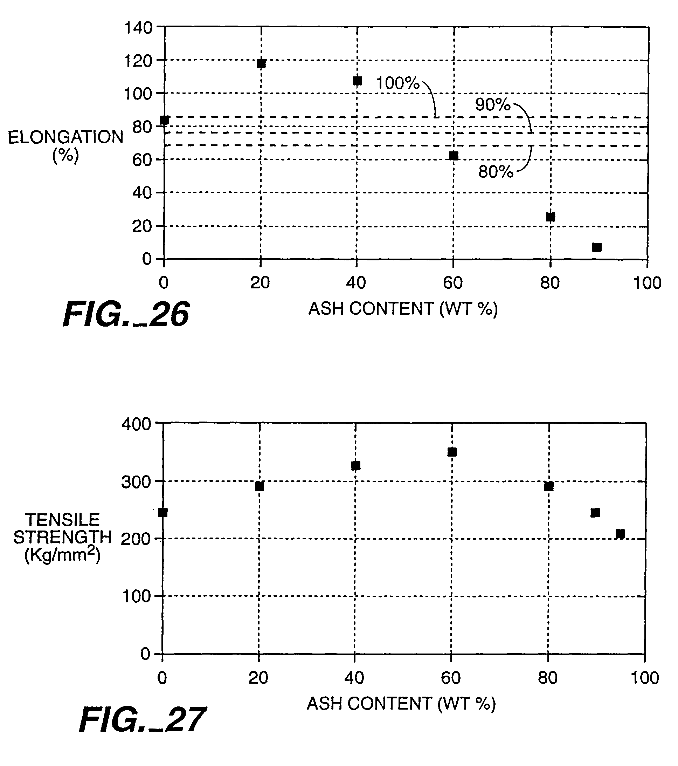

The invention further relates to cloth materials having organic ashes mixed with the fibers by 0.01% to 50%. Desired effects cannot be obtained if the ash content is less than 0.01%. If too much ashes are added to the resin material from which fibers are produced, however, the strength of the produced fibers is adversely affected. One of representative physical characteristics of fibers affected by the addition of organic ashes according to this invention is the elongation, defined as the fractional change in length when the fiber is subjected to a tensile force until it breaks. In order to investigate the effect of newspaper ashes on the elongation of fibers, a test of conducted by preparing sample fibers with newspaper ashes mixed into polyethylene terephthalate (PET) resin material by 0%, 20%, 40%, 60%, 80% and 90% and spinning under the conditions with extrusion rate of 2.5g/min, spinning temperature of 295°C and nozzle diameter of 0.3mm. The results of the test are shown in Fig. 26, that is, the elongation increases as the ash content is increased, reaches a maximum value when the ash content is about 20%, and drops thereafter such that the fibers break easily and cease to be useful if the ash content exceeds 90%. The contents of newspaper ashes, at which the fiber elongation of the sample became 90% and 80% of the ash-free sample, were respectively 55% and 58%.

Effects, similar to those obtained in Test No. 11, could be obtained by using cloth materials produced with fibers (inclusive of chemical fibers) containing seaweed ashes by 5%. Similar effects were obtained by using cloth materials coated with a coating material containing seaweed ashes, those processed with a resin material containing seaweed ashes, or those to which seaweed ashes mixed in a glue have been applied.

### Test No. 13 (Cosmetic articles)

This invention also relates to cosmetic articles such as packs containing organic ashes by 0.01% to 40%. Desired effects cannot be obtained if the ash content is less than 0.01%. In order to keep the pack elastic, ash contents up to 40% are preferred.

As an example, cosmetic packs were produced by mixing organic ashes to the pack material or to the additive to the material by 2%. By applying such packs to the skin, a significant amount of far infrared radiation could be obtained at body temperatures with effects such as acceleration of metabolism.

In another test, cosmetic packs and liquids containing ashes of defatted soy beams by 1% were used and it was observed that freckles and stains on the skin of patients slowly disappeared in 5 months and that they completely disappeared in about a year. This was probably because blood circulation was improved by far infrared radiation.

In still another test, hair brushes and combs were produced with seaweed ashes mixed at a rate of 5%. Thirteen months after a person serving as test subject began using them, it was microscopically ascertained that the cuticles of the hair were smoother. Similar effects were observed about 3 months after a person began using a hair brush and a comb containing seaweed ashes by 50%. Effects are evidently greater if the content of seaweed ashes is higher.

### Test No. 14 (Enamel materials)

This invention also relates to enamel materials containing organic ashes by 0.01% to 50%. If the ash content is less than 0.01%, no desirable effect can be obtained. If the ash content is greater than 50%, problems arise in the material strength.

If ashes of defatted edible soy beans are added to the raw material for an enamel or the additive therefor by 0.01% to make a bathtub or a toilet seat by firing, or if seaweed ashes are added to the substance which is attached or adsorbed thereto, the bath tub thus produced has an energy-conserving quality because it holds heat well and the toilet seat thus produced is more comfortable because of its heat-retaining property.

### Test No. 15 (Ceramics and glass materials)

The invention also relates to ceramics made from a material containing organic ashes. One of the physical characteristics of ceramic materials which are significantly affected by the temperature of the final heating process is the tensile strength. Compressive and bending strengths are said to be not significantly affected. In order to ascertain the effect of ash content on the tensile strength of ceramic materials, test samples were prepared by heating potassium feldspar to 1500°C and cooling it suddenly in water to pulverize it. Sardine ashes were then mixed in at weight ratios of 0%, 20%, 40%, 80%, 90% and 95% and sample pieces of 1cm x 1cm x 10cm were prepared by firing at 950°C. As shown in Fig. 27, the tensile strength was 345kg/cm² and at its maximum when the sardine ash content was 60%, the same at 90% as when no ashes were added (content of 0%) and lower at 95% than when no ashes were added.

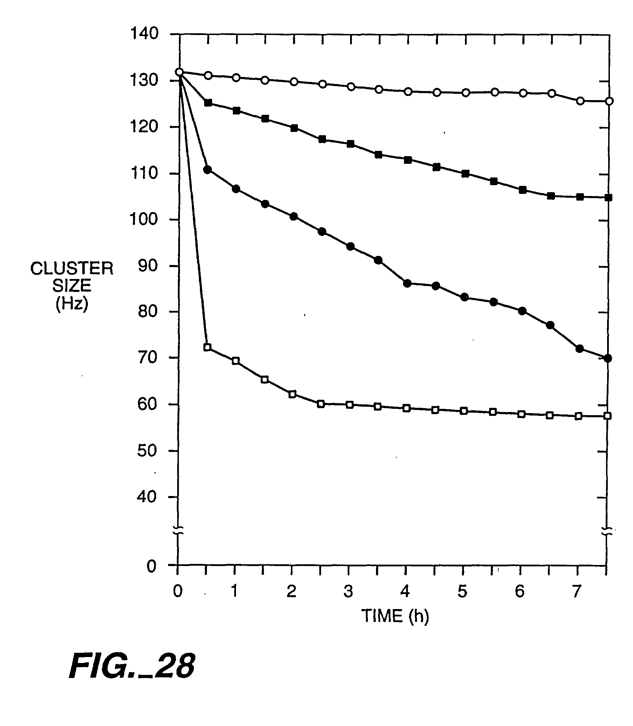

As another test, flower vases were produced by mixing sardine ashes into a ceramic material by 0.005%, 0.01%, 50% and 90% and tap water with cluster size of 132Hz was poured therein. The subsequent changes in the cluster size of the water were studied by nuclear magnetic resonance spectroscopy. The results are shown in Fig. 28. It can be seen that the cluster size of water can be reduced sufficiently if the ash content is 0.1% to 90%. The effects were negligible if the ash content was 0.005%. Similar results were also obtained by using glass jars containing sardine ashes or organic ashes of other kinds.

Flowers put in a glass vase containing sardine ashes by 50% or a porcelain made by mixing sardine ashes by 50% to the clay material lasted for 10 days while flowers put in vases not containing ashes lasted only 4 to 5 days. If such ash-containing materials are used to make a bottle, the taste of the rice wine kept therein became milder.

The invention has been described above by way of many examples but these examples are intended to be illustrative, not limiting. Many useful applications are conceivable where far infrared radiation is known to be, or can be suspected to be, beneficial. The specification is intended to be interpreted broadly. For example, construction materials and household items are intended to include not only dinnerware but also a wider variety of items such as vehicular accessories, gardening tools, bedding and furniture. All modification and variation of the disclosure that may be apparent to a person skilled in the art are intended to be within the scope of this invention.

## Claims

1. A radiation-emitting product which is free from carbon and comprises a base material and at least 0.01 weight % of organic ashes, said organic ashes having emissivity greater than 60% of far infrared radiation of wavelengths 8-12*µ*m at human body temperatures.

2. The product of claim 1 capable of serving as an ointment, containing said organic ashes by 0.01 - 30 weight %, said base material being a medicament.

3. The product of claim 1 capable of serving as a cataplasm poultice, containing said organic ashes by 0.01 - 20 weight %, said base material being a medicament.

4. The product of claim 1 capable of serving as an adhesive tape for medical use, containing said organic ashes by 0.01 - 20 weight %, said base material including a base and an adhesive material attached to said base.

5. The product of claim 1 wherein said base material is a resin, the content of said organic ashes in said base material being such that the tensile strength of said base material with said organic ashes is greater than 80% of the tensile strength of said base material without containing any of said organic ashes.

6. The product of claim 1 wherein said base material is synthetic fibers, the content of said organic ashes in said base material being such that the elongation of said synthetic fibers with said organic ashes is greater than 80% of the elongation of said base material without containing any of said organic ashes.

7. The product of claim 1 wherein said base material is a coating material, the content of said organic ashes in said base material being such that the adhesion strength of said base material with said organic ashes is greater than 80% of the adhesion strength of said base material without any of said organic ashes.

8. The product of claim 1 containing said organic ashes by 0.01 - 30 weight %, said base material comprising paper material.

9. The product of claim 1 capable of serving as a cosmetic product, containing said organic ashes by 0.01 - 40 weight %, said base material comprising a medicament.

10. The product of claim 1 capable of serving as an accessory to be worn by a person and further comprising a main body, said base material being a resin containing said organic ashes by at least 0.01 weight % and being buried inside said main body.

11. The product of claim 1 capable of serving as an accessory to be worn by a person and further comprising a main body, said base material being a coating material containing said organic ashes by at least 0.01 weight % and being attached to said main body by firing.

12. The product of claim 1 which is an inorganic substance comprising glass, said glass containing said organic ashes by 0.01 - 70 weight %.

13. The product of claim 1 which is an inorganic substance comprising a ceramic, said ceramic containing said organic ashes by 0.01 - 90 weight %.

14. The product of claim 1 wherein said organic ashes are coated over said base material.

15. The product of claim 1 wherein said organic ashes are mixed in said base material, said product being capable of serving as at least one selected from the group consisting of vehicular accessories, dinnerware, gardening tools, bedding and furniture.

16. The product of claim 15 containing said organic ashes by 0.01 - 30 weight %, said product comprising paper.

17. The product of claim 15 which is capable of serving as a coating material, said base material containing said organic ashes by 0.01 - 90 weight %.

18. The product of claim 15 containing said organic ashes by 0.01 - 50 weight %, said base material comprising fibers, said product being capable of serving as a cloth material.

19. The product of claim 1 capable of serving as a cloth material, said base material comprising synthetic fibers.

20. The product of claim 1 capable of serving as a coating material, said base material comprising resins.

21. A method of manufacturing a composition being capable of applying far infrared radiation at temperatures no higher than the human body temperature, said method comprising the steps of:
obtaining carbon-free organic ashes having emissivity greater than 60% of far infrared radiation of wavelengths 8-12 µm at human body temperatures by completely combusting organic materials selected from the group consisting of animals, birds, fishes, shells, organs and excrements thereof, seaweeds, plants, woods and grains, and thereafter removing carbon;
adding said organic ashes to a base material to produce a far-infrared radiation emitting product.

22. The method of claim 21 wherein said far-infrared radiation emitting product is capable of serving as an ointment and contains said organic ashes by 0.01 - 30 weight %, said base material being a medicament.

23. The method of claim 21 wherein said far-infrared radiation emitting product is capable of serving as a cataplasm poultice and contains said organic ashes by 0.01 - 20 weight %, said base material being a medicament.

24. The method of claim 21 wherein said far-infrared radiation emitting product is capable of serving as an adhesive tape for medical use and contains said organic ashes by 0.01 - 20 weight %, said base material including a base and an adhesive material attached to said base.

25. The method of claim 21 wherein said base material is a resin, the content of said organic ashes in said base material being such that the tensile strength of said base material with said organic ashes is greater than 80% of the tensile strength of said base material without containing any of said organic ashes.

26. The method of claim 21 wherein said base material is synthetic fibers, the content of said organic ashes in said base material being such that the elongation of said synthetic fibers with said organic ashes is greater than 80% of the elongation of said base material without containing any of said organic ashes.

27. The method of claim 21 wherein said base material is a coating material, the content of said organic ashes in said base material being such that the adhesion strength of said base material with said organic ashes is greater than 80% of the adhesion strength of said base material without any of said organic ashes.

28. The method of claim 21 wherein said far-infrared radiation emitting product contains said organic ashes by 0.01 - 30 weight %, said base material comprising paper material.

29. The method of claim 21 wherein said far-infrared radiation emitting product is capable of serving as a cosmetic product and contains said organic ashes by 0.01 - 40 weight %, said base material comprising a medicament.

30. The method of claim 21 wherein said far-infrared radiation emitting product further comprises a main body and is capable of serving as an accessory to be worn by a person, said base material being a resin containing said organic ashes at least by 0.01 weight %, said method further comprising the step of burying said base material containing said organic ashes inside said main body.

31. The method of claim 21 wherein said far-infrared radiation emitting product further comprises a main body and is capable of serving as an accessory to be worn by a person, said base material being a coating material containing said organic ashes at least by 0.01 weight %, said method further comprising the step of attaching said base material containing said organic ashes on said main body by firing.

32. The method of claim 21 wherein said far-infrared radiation emitting product is an inorganic substance comprising glass, said glass containing said organic ashes by 0.01 - 70 weight %.

33. The method of claim 21 wherein said far-infrared radiation emitting product is an inorganic substance comprising a ceramic, said ceramic containing said organic ashes by 0.01 - 90 weight %.

34. The method of claim 21 wherein said step of adding said organic ashes comprises coating said organic ashes over said base material.

35. The method of claim 21 wherein said step of adding said organic ashes comprises mixing said organic ashes in said base material, said product being capable of serving as at least one selected from the group consisting of vehicular accessories, dinnerware, gardening tools, bedding and furniture.

36. The method of claim 35 wherein said far-infrared radiation emitting product comprises paper containing said organic ashes by 0.01 - 30 weight %.

37. The method of claim 35 wherein said far-infrared radiation emitting product contains said organic ashes by 0.01 - 90 weight % and is capable of serving as a coating material.

38. The method of claim 35 wherein said far-infrared radiation emitting product contains said organic ashes by 0.01 - 50 weight % and is capable of serving as a cloth material, said base material comprising chemical fibers.

39. The method of claim 21 wherein said far-infrared radiation emitting product is capable of serving as a cloth material, said base material comprising synthetic fibers.

40. The method of claim 21 wherein said far-infrared radiation emitting product is capable of serving as a coating material, said base material comprising resins.

## Patentansprüche

1. Kohlenstofffreies strahlungsemittierendes Produkt, umfassend ein Basismaterial und wenigstens 0,01 Gew.-% organischer Aschen, wobei die organischen Aschen ein Emissionsvermögen von größer 60% an ferner Infrarotstrahlung mit Wellenlängen von 8 bis 12 µm bei Temperaturen des menschlichen Körpers besitzen.

2. Produkt nach Anspruch 1 für eine Salbe, enthaltend die organischen Aschen mit 0,01 bis 30 Gew.-%, wobei das Basismaterial ein Medikament ist.

3. Produkt nach Anspruch 1 für einen Brei-Umschlag, enthaltend die organischen Aschen mit 0,01 bis 20 Gew.-%, wobei das Basismaterial ein Medikament ist.

4. Produkt nach Anspruch 1 für ein Klebeband für medizinische Verwendung, enthaltend die organischen Aschen mit 0,01 bis 20 Gew.-%, wobei das Basismaterial eine Basis und ein an diese Basis befestigtes Klebematerial einschließt.

5. Produkt nach Anspruch 1, worin das Basismaterial ein Harz ist, und der Gehalt an organischen Aschen im Basismaterial derart ist, dass die Zugfestigkeit des Basismaterials mit den organischen Aschen größer ist als 80% der Zugfestigkeit des Basismaterials, welches keine organischen Aschen enthält.

6. Produkt nach Anspruch 1, worin das Basismaterial aus synthetischen Fasern besteht, und der Gehalt der organischen Aschen im Basismaterial derart ist, dass die Dehnung der synthetischen Fasern mit den organischen Aschen größer ist als 80% der Dehnung des Basismaterials, welches keine der organischen Aschen enthält.

7. Produkt nach Anspruch 1, worin das Basismaterial ein Beschichtungsmaterial ist, und der Gehalt der organischen Aschen im Basismaterial derart ist, dass die Adhäsionsfestigkeit des Basismaterials mit den organische Aschen größer ist als 80% der Adhäsionsfestigkeit des Basismaterials, welches keine der organischen Aschen enthält.

8. Produkt nach Anspruch 1, enthaltend die organischen Aschen mit 0,01 bis 30 Gew.-%, wobei das Basismaterial ein Papiermaterial umfasst.

9. Produkt nach Anspruch 1 für ein kosmetisches Produkt, enthaltend die organischen Aschen mit 0,01 bis 40 Gew.-%, wobei das Basismaterial ein Medikament umfasst.

10. Produkt nach Anspruch 1 für ein durch eine Person zu tragendes Accessoire, umfassend weiterhin einen Hauptkörper, wobei das Basismaterial ein Harz ist, enthaltend die organischen Aschen mit wenigstens 0,01 Gew.-%, und welches innerhalb des Hauptkörpers eingebettet ist.

11. Produkt nach Anspruch 1 für ein durch eine Person zu tragendes Accessiore, umfassend weiterhin einen Hauptkörper, wobei das Basismaterial ein Beschichtungsmaterial ist, enthaltend die organischen Aschen mit wenigstens 0,01 Gew.-%, und welches an den Hauptkörper durch Hartbrennen befestigt ist.

12. Produkt nach Anspruch 1, welches eine organische Substanz ist, umfassend Glas, wobei das Glas die organischen Aschen mit 0,01 bis 70 Gew.-% enthält.

13. Produkt nach Anspruch 1, welches eine anorganische Substanz ist, umfassend eine Keramik, wobei die Keramik die organischen Aschen mit 0,01 bis 90 Gew.-% enthält.

14. Produkt nach Anspruch 1, worin die organischen Aschen über das Basismaterial beschichtet sind.

15. Produkt nach Anspruch 1, worin die organischen Aschen mit dem Basismaterial gemischt sind, wobei das Produkt für wenigstens ein Mitglied, ausgewählt aus der Gruppe, bestehend aus Fahrzeugaccessoires, Geschirrwaren, Gartenwerkzeugen, Bettzeug und Möbel, einsetzbar ist.

16. Produkt nach Anspruch 15, enthaltend die organischen Aschen mit 0,01 bis 30 Gew.-%, wobei das Produkt Papier umfasst.

17. Produkt nach Anspruch 15 für ein Beschichtungsmaterial, wobei das Basismaterial die organischen Aschen mit 0,01 bis 90 Gew.-% enthält.

18. Produkt nach Anspruch 15, enthaltend die organischen Aschen mit 0,01 bis 50 Gew.-%, wobei das Basismaterial Fasern umfasst, und das Produkt für Tuch einsetzbar ist.

19. Produkt nach Anspruch 1 für Kleidungsmaterial, wobei das Basismaterial synthetische Fasern umfasst.

20. Produkt nach Anspruch 1 für ein Beschichtungsmaterial, wobei das Basismaterial Harze umfasst.

21. Verfahren zur Herstellung einer Zusammensetzung, welche fähig ist, Strahlung im fernen Infrarot anzuwenden bei Temperaturen, die nicht höher sind als die Temperatur des menschlichen Körpers, wobei das Verfahren die Schritte umfasst:
Erhalten kohlenstofffreier organischer Aschen mit einem Emissionsvermögen von größer 60% Strahlung im fernen Infrarot mit Wellenlängen von 8 bis 12 µm bei Temperaturen des menschlichen Körpers, durch vollständiges Verbrennen organischer Materialien, ausgewählt aus der Gruppe, bestehend aus Tieren, Vögeln, Fischen, Schalentieren, Organen und Exkrementen davon, Seetang, Pflanzen, Hölzern und Körnern, und anschließendes Entfernen von Kohlenstoff;
Zugeben der organischen Aschen zu einem Basismaterial zur Herstellung eines im fernen Infrarot Strahlung emittierenden Produkts.

22. Verfahren nach Anspruch 21, worin das im fernen Infrarot Strahlung emittierende Produkt für eine Salbe einsetzbar ist und die organischen Aschen mit 0,01 bis 30 Gew.-% enthält, wobei das Basismaterial ein Medikament ist.

23. Verfahren nach Anspruch 21, worin das im fernen Infrarot Strahlung emittierende Produkt für einen Brei-Umschlag verwendbar ist und die organischen Aschen mit 0,01 bis 20 Gew.-% enthält, wobei das Basismaterial ein Medikament ist.

24. Verfahren nach Anspruch 21, worin das im fernen Infrarot Strahlung emittierende Produkt als Klebeband für medizinische Verwendung einsetzbar ist und die organischen Aschen mit 0,01 bis 20 Gew.-% enthält, wobei das Basismaterial eine Basis und ein an diese Basis befestigtes Klebematerial einschließt.

25. Verfahren nach Anspruch 21, worin das Basismaterial ein Harz ist, und der Gehalt der organischen Aschen in dem Basismaterial derart ist, dass die Zugfestigkeit des Basismaterials mit organischen Aschen größer als 80% der Zugfestigkeit des Basismaterials ist, welches keine der organischen Aschen enthält.

26. Verfahren nach Anspruch 21, worin das Basismaterial aus synthetischen Fasern besteht, und der Gehalt der organischen Aschen in dem Basismaterial derart ist, dass die Dehnung der synthetischen Fasern mit organischen Aschen größer als 80% der Dehnung des Basismaterials ist, welches keine der organischen Aschen enthält.

27. Verfahren nach Anspruch 21, worin das Basismaterial ein Beschichtungsmaterial ist, und der Gehalt der organischen Aschen in dem Basismaterial derart ist, dass die Adhäsionsfestigkeit des Basismaterials mit den organischen Aschen größer als 80% der Adhäsionsfestigkeit des Basismaterials ist, welches keine der organischen Aschen enthält.

28. Verfahren nach Anspruch 21, worin das im fernen Infrarot Strahlung emittierende Produkt die organischen Aschen mit 0,01 bis 30 Gew.-% enthält, wobei das Basismaterial Papiermaterial umfasst.

29. Verfahren nach Anspruch 21, worin das im fernen Infrarot Strahlung emittierende Produkt für ein kosmetisches Produkt einsetzbar ist und die organischen Aschen mit 0,01 bis 40 Gew.-% enthält, wobei das Basismaterial ein Medikament umfasst.

30. Verfahren nach Anspruch 21, worin das im fernen Infrarot Strahlung emittierende Produkt weiterhin einen Hauptkörper umfasst und als von einer Person zu tragendes Zusatzteil einsetzbar ist, wobei das Basismaterial ein Harz ist, enthaltend die organischen Aschen mit wenigstens 0,01 Gew.-%, wobei das Verfahren weiterhin den Schritt des Einbettens des die organischen Aschen enthaltenden Basismaterials in den Hauptkörper umfasst.

31. Verfahren nach Anspruch 21, worin das im fernen Infrarot Strahlung emittierende Produkt weiterhin einen Hauptkörper umfasst und als von einer Person zu tragendes Zusatzteil einsetzbar ist, wobei das Basismaterial ein Beschichtungsmaterial ist, welches die organischen Aschen mit wenigstens 0,01 Gew.-% enthält, wobei das Verfahren weiterhin den Schritt des Befestigens des die organischen Aschen enthaltenden Basismaterials auf dem Hauptkörper mittels Hartbrennen umfasst.

32. Verfahren nach Anspruch 21, worin das im fernen Infrarot Strahlung emittierende Produkt eine anorganische Substanz ist, umfassend Glas, wobei das Glas die organischen Aschen mit 0,01 bis 70 Gew.-% enthält.

33. Verfahren nach Anspruch 21, worin das im fernen Infrarot Strahlung emittierende Produkt eine anorganische Substanz ist, umfassend eine Keramik, wobei die Keramik die organischen Aschen mit 0,01 bis 90 Gew.-% enthält.

34. Verfahren nach Anspruch 21, worin der Schritt des Zugebens der organischen Aschen das Beschichten der organischen Aschen über das Basismaterial umfasst.

35. Verfahren nach Anspruch 21, worin der Schritt des Zugebens der organischen Aschen das Mischen der organischen Aschen in dem Basismaterial umfasst, wobei das Produkt für wenigstens ein Mitglied, ausgewählt aus der Gruppe, bestehend aus Fahrzeugzusatzteilen, Geschirrware, Gartenwerkzeuge, Bettzeug und Möbel, einsetzbar ist.

36. Verfahren nach Anspruch 35, worin das im fernen Infrarot Strahlung emittierende Produkt Papier umfasst, welches die organischen Aschen mit 0,01 bis 30 Gew.-% enthält.

37. Verfahren nach Anspruch 35, worin das im fernen Infrarot Strahlung emittierende Produkt die organischen Aschen mit 0,01 bis 90 Gew.-% enthält und als Beschichtungsmaterial einsetzbar ist.

38. Verfahren nach Anspruch 35, worin das im fernen Infrarot Strahlung emittierende Produkt die organischen Aschen mit 0,01 bis 50 Gew.-% enthält und als Kleidungsmaterial einsetzbar ist, wobei das Basismaterial chemische Fasern umfasst.

39. Verfahren nach Anspruch 21, worin das im fernen Infrarot Strahlung emittierende Produkt für Tuch einsetzbar ist, wobei das Basismaterial synthetische Fasern umfasst.

40. Verfahren nach Anspruch 21, worin das im fernen Infrarot Strahlung emittierende Produkt für Beschichtungsmaterial einsetzbar ist, wobei das Basismaterial Harze umfasst.

## Revendications

1. Produit émettant un rayonnement, ne contenant pas de carbone et comprenant un matériau de base et au moins 0,01% en poids de cendres organiques, lesdites cendres organiques ayant un pouvoir émissif supérieur à 60% d'un rayonnement infrarouge lointain de longueurs d'onde 8 - 12 µm aux températures corporelles humaines.

2. Produit selon la revendication 1, susceptible de servir de pommade, contenant lesdites cendres organiques à raison de 0,01 - 30% en poids, ledit matériau de base étant un médicament.

3. Produit selon la revendication 1, susceptible de servir de cataplasme, contenant lesdites cendres organiques à raison de 0,01 - 20% en poids, ledit matériau de base étant un médicament.

4. Produit selon la revendication 1, capable de servir de ruban adhésif à usage médical, contenant lesdites cendres organiques à raison de 0,01 - 20% en poids, ledit matériau de base comprenant une base et un matériau adhésif fixé à ladite base.

5. Produit selon la revendication 1, dans lequel ledit matériau de base est une résine, la teneur en lesdites cendres organiques dudit matériau de base étant telle que la résistance à la traction dudit matériau de base avec lesdites cendres organiques soit supérieure à 80% de la résistance à la traction dudit matériau de base ne contenant pas lesdites cendres organiques.

6. Produit selon la revendication 1, dans lequel ledit matériau de base est des fibres synthétiques, la teneur en lesdites cendres organiques dudit matériau de base étant telle que l'allongement desdites fibres synthétiques avec lesdites cendres organiques soit supérieur à 80% de l'allongement dudit matériau de base ne contenant pas lesdites cendres organiques.

7. Produit selon la revendication 1, dans lequel ledit matériau de base est un matériau de revêtement, la teneur en lesdites cendres organiques dudit matériau de base étant telle que l'adhérence dudit matériau de base avec lesdites cendres organiques soit supérieure à 80% de l'adhérence dudit matériau de base ne contenant pas lesdites cendres organiques.

8. Produit selon la revendication 1, contenant lesdites cendres organiques à raison de 0,01 - 30% en poids, ledit matériau de base comprenant un matériau de papier.

9. Produit selon la revendication 1 susceptible de servir de produit cosmétique, contenant lesdites cendres organiques à raison de 0,01 - 40% en poids, ledit matériau de base comprenant un médicament.

10. Produit selon la revendication 1 susceptible de servir d'accessoire à porter par une personne et comprenant en outre un corps principal, ledit matériau de base étant une résine contenant au moins 0,01% en poids desdites cendres organiques et étant noyé à l'intérieur dudit corps principal.

11. Produit selon la revendication 1 susceptible de servir d'accessoire à porter par une personne et comprenant en outre un corps principal, ledit matériau de base étant un matériau de revêtement contenant au moins 0,01% en poids desdites cendres organiques et étant fixé audit corps principal par cuisson.

12. Produit selon la revendication 1, qui est une substance minérale comprenant du verre, ledit verre contenant lesdites cendres organiques à raison de 0,01 - 70% en poids.

13. Produit selon la revendication 1, qui est une substance minérale comprenant une céramique, ladite céramique contenant lesdites cendres organiques à raison de 0,01 - 90% en poids.

14. Produit selon la revendication 1, dans lequel lesdites cendres organiques sont appliquées comme revêtement sur ledit matériau de base.

15. Produit selon la revendication 1, dans lequel lesdites cendres organiques sont mélangées dans ledit matériau de base, ledit produit étant susceptible de servir au moins d'accessoires automobile, de vaisselle, d'outil de jardinage, de literie et de meubles.

16. Produit selon la revendication 15, contenant lesdites cendres organiques à raison de 0,01 - 30% en poids, ledit produit comprenant du papier.

17. Produit selon la revendication 15 susceptible de servir de matériau de revêtement, ledit matériau de base contenant lesdites cendres organiques à raison de 0,01 - 90% en poids.

18. Produit selon la revendication 15 contenant lesdites cendres organiques à raison de 0,01 - 50% en poids, ledit matériau de base comprenant des fibres, ledit produit étant susceptible de servir de tissu.

19. Produit selon la revendication 1 susceptible de servir de tissu, ledit matériau de base comprenant des fibres synthétiques.

20. Produit selon la revendication 1 susceptible de servir de matériau de revêtement, ledit matériau de base comprenant des résines.

21. Procédé de fabrication d'une composition susceptible d'appliquer un rayonnement infrarouge lointain à des températures non supérieures à la température corporelle humaine, ledit procédé comprenant les étapes consistant à:
obtenir des cendres organiques sans carbone ayant un pouvoir émissif supérieur à 60% d'un rayonnement infrarouge lointain de longueurs d'onde 8 - 12 µm aux températures corporelles humaines, en brûlant complètement des matériaux organiques choisis dans le groupe constitué par les animaux, les oiseaux, les poissons, les coquillages, leurs organes et leurs excréments, les algues marines, les végétaux, les bois et les grains, puis en éliminant le carbone;
ajouter lesdites cendres organiques à un matériau de base pour produire un produit émettant un rayonnement infrarouge lointain.

22. Procédé selon la revendication 21, dans lequel ledit produit émettant un rayonnement infrarouge lointain est susceptible de servir de pommade et contient lesdites cendres organiques à raison de 0,01 - 30% en poids, ledit matériau de base étant un médicament.

23. Procédé selon la revendication 21, dans lequel ledit produit émettant un rayonnement infrarouge lointain est susceptible de servir de cataplasme et contient lesdites cendres organiques à raison de 0,01 - 20% en poids, ledit matériau de base étant un médicament.

24. Procédé selon la revendication 21, dans lequel ledit produit émettant un rayonnement infrarouge lointain est susceptible de servir de ruban adhésif à usage médical et contient lesdites cendres organiques à raison de 0,01 - 20% en poids, ledit matériau de base contenant une base et un matériau adhésif fixé à ladite base.

25. Procédé selon la revendication 21, dans lequel ledit matériau de base est une résine, la teneur en lesdites cendres organiques dudit matériau de base étant telle que la résistance à la traction dudit matériau de base avec lesdites cendres organiques soit supérieure à 80% de la résistance à la traction dudit matériau de base ne contenant pas lesdites cendres organiques.

26. Procédé selon la revendication 21, dans lequel ledit matériau de base est des fibres synthétiques, la teneur en lesdites cendres organiques dudit matériau de base étant telle que l'allongement desdites fibres synthétiques avec lesdites cendres organiques soit supérieur à 80% de l'allongement dudit matériau de base ne contenant pas lesdites cendres organiques.

27. Procédé selon la revendication 21, dans lequel ledit matériau de base est un matériau de revêtement, la teneur en lesdites cendres organiques dudit matériau de revêtement étant telle que l'adhérence dudit matériau de base avec lesdites cendres organiques soit supérieure à 80% de l'adhérence dudit matériau de base ne contenant pas lesdites cendres organiques.

28. Procédé selon la revendication 21 dans lequel ledit produit émettant un rayonnement infrarouge lointain contient lesdites cendres organiques à raison de 0,01 - 30% en poids, ledit matériau de base comprenant un matériau de papier.

29. Procédé selon la revendication 21 dans lequel ledit produit émettant un rayonnement infrarouge lointain est susceptible de servir de produit cosmétique et contient lesdites cendres organiques à raison de 0,01 - 40% en poids, ledit matériau de base comprenant un médicament.

30. Procédé selon la revendication 21 dans lequel ledit produit émettant un rayonnement infrarouge lointain comprend par ailleurs un corps principal et est susceptible de servir d'accessoire destiné à être porté par une personne, ledit matériau de base étant une résine contenant au moins 0,01% en poids desdites cendres organiques, ledit procédé comprenant en outre l'étape consistant à noyer ledit matériau de base contenant lesdites cendres organiques à l'intérieur dudit corps principal.

31. Procédé selon la revendication 21 dans lequel ledit produit émettant un rayonnement infrarouge lointain comprend en outre un corps principal et est capable de servir d'accessoire destiné à être porté par une personne, ledit matériau de base étant un matériau de revêtement contenant au moins 0,01% en poids desdites cendres organiques, ledit procédé comprenant en outre l'étape consistant à fixer ledit matériau de base contenant lesdites cendres organiques sur ledit corps principal par cuisson.

32. Procédé selon la revendication 21 dans lequel ledit produit émettant un rayonnement infrarouge lointain est une substance minérale comprenant du verre, ledit verre contenant lesdites cendres organiques à raison de 0,01 - 70% en poids.

33. Procédé selon la revendication 21 dans lequel ledit produit émettant un rayonnement infrarouge lointain est une substance minérale comprenant une céramique, ladite céramique contenant lesdites cendres organiques à raison de 0,01 - 90% en poids.

34. Procédé selon la revendication 21 dans lequel ladite étape d'addition desdites cendres organiques comprend l'application d'un revêtement desdites cendres organiques sur ledit matériau de base.

35. Procédé selon la revendication 21 dans lequel ladite étape d'addition desdites cendres organiques comprend le mélange desdites cendres organiques dans ledit matériau de base, ledit produit étant susceptible de servir au moins d'accessoire automobile, de vaisselle, d'outil de jardinage, de literie et de meuble.

36. Procédé selon la revendication 35 dans lequel ledit produit émettant un rayonnement infrarouge lointain comprend du papier contenant lesdites cendres organiques à raison de 0,01 - 30% en poids.

37. Procédé selon la revendication 35 dans lequel ledit produit émettant un rayonnement infrarouge lointain contient lesdites cendres organiques à raison de 0,01 - 90% en poids et est susceptible de servir de matériau de revêtement.

38. Procédé selon la revendication 35 dans lequel ledit produit émettant un rayonnement infrarouge lointain contient lesdites cendres organiques à raison de 0,01 - 50% en poids et est susceptible de servir de tissu, ledit matériau de base comprenant des fibres chimiques.

39. Procédé selon la revendication 21 dans lequel ledit produit émettant un rayonnement infrarouge lointain est susceptible de servir de tissu, ledit matériau de base comprenant des fibres synthétiques.

40. Procédé selon la revendication 21 dans lequel ledit produit émettant un rayonnement infrarouge lointain est susceptible de servir de matériau de revêtement, ledit matériau de base comprenant des résines.
